# EUROPEAN PATENT APPLICATION

(11) **EP 2 887 414 A1**
(43) Date of publication of application: **24.06.2015**
(21) Application number: 13829927.6
(22) Date of filing: 15.08.2013
(51) Int. Cl.: H01L 51/30, C07D 495/04, C07D 495/18, C08L 101/00, H01L 29/786, H01L 51/05, H01L 51/40

(54) **ORGANIC SEMICONDUCTOR SOLUTION AND ORGANIC SEMICONDUCTOR FILM**

(30) Priority: 15.08.2012 JP 2012180177; 29.03.2013 JP 2013073463
(71) Applicant: Teijin Limited, Osaka-shi, Osaka 541-0054 (JP)
(72) Inventor: IKEDA, Yoshinori, Hino-shi Tokyo 191-0065 (JP); KOHNO, Azusa, Hino-shi Tokyo 191-0065 (JP)
(74) Representative: Carpmaels & Ransford LLP
(86) International application number: PCT/JP2013/071982
(87) International publication number: WO 2014/027685

(57) **Abstract**

The present invention provides an organic semiconductor solution that can produce an organic semiconductor film having acceptable semiconductor properties while endowed with improved film-forming properties due to substantial polymer content. This organic semiconductor solution contains an organic solvent as well as a polymer and an organic semiconductor precursor dissolved in the organic solvent. The proportion of the polymer in relation to the total of the polymer and the organic semiconductor precursor is equal to or greater than 3 mass%. The organic semiconductor precursor has a structure in which a dienophile-type alkene is eliminably bonded through a double bond thereof to the benzene ring of an organic semiconductor compound represented by formula I) (where A₁ to A₈ and E₁ to E₄ are independently selected from the group consisting of hydrogen atoms, halogen atoms, C₁ to C₂₀ alkyl groups, and the like, and Y represents sulfur or selenium).

## Description

### TECHNICAL FIELD

The present invention relates to an organic semiconductor solution for use in the formation of an organic semiconductor film, and a use method thereof. The present invention also relates to an organic semiconductor film obtained using the organic semiconductor solution.

### BACKGROUND ART

With regard to a semiconductor compound, various studies are being made on its utilization in an organic thin-film transistor (TFT), an organic carrier transport layer, or an organic semiconductor layer for an organic light-emitting device, etc. In particular, a thin-film transistor having an organic semiconductor layer composed of an organic semiconductor compound is expected, as a low-cost and lightweight device, to substitute for the current silicon-based transistor. The organic semiconductor layer is also expected to find its application to a smart tag, a lightweight display, etc. by utilizing the advantages peculiar to an organic material, such as lightness and flexibility.

Among these organic semiconductor compounds, a condensed polycyclic aromatic compound, particularly, a condensed polycyclic aromatic compound in which aromatic rings are linearly or crookedly connected as in pentacene of the following formula, has become found to be preferred in terms of semiconductor characteristics such as mobility of the carrier:

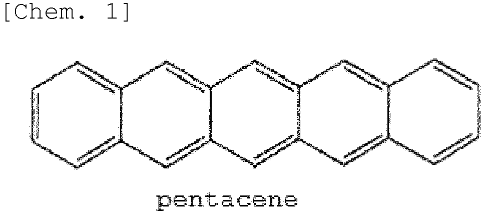

Such a condensed polycyclic aromatic compound has high aromaticity and high crystallinity and in turn, exhibits very low solubility in an organic solvent, etc., making it difficult to use the compound in a coating method. For this reason, in the case of obtaining an organic semiconductor film by using a condensed polycyclic aromatic compound, it is a general practice to employ a vapor deposition method.

However, the production of a semiconductor film by a solution method is preferred in view of the production efficiency, required production facilities, etc., and therefore attempts are being made to increase the solubility of a condensed polycyclic aromatic compound in an organic solvent, etc. and thereby enable the film formation of a condensed aromatic compound by a solution method. In this regard, for example, introduction of a substituent into a condensed polycyclic aromatic compound so as to increase the solubility has been proposed (Patent Documents 1 and 2).

It has been also proposed to introduce a releasable substituent into a condensed polycyclic aromatic compound, thereby obtaining a condensed polycyclic aromatic compound precursor with an increased solubility, dissolve the precursor in an organic solvent, and after film formation, eliminate the substituent by heating and/or light irradiation to obtain an organic semiconductor film (Patent Documents 3 and 4).

Furthermore, it has been proposed to improve the film formability of an organic semiconductor solution by dissolving a semiconducting or insulating polymer in an organic solvent together with the above-described polycondensed polycyclic aromatic compound increased in the solubility (Patent Documents 5 and 6).

Specifically, in Patent Document 5, an organic semiconductor film is obtained using an organic semiconductor solution prepared by dissolving an organic semiconductor compound precursor and a polymer in an organic solvent.

However, in the case of using an organic semiconductor compound precursor as in Patent Document 5, there is a problem that when the polymer concentration is increased, the semiconductor characteristics of the obtained organic semiconductor film are significantly deteriorated. Specifically, in examples of Patent Document 5, it is revealed that in the case of using an organic semiconductor solution obtained by dissolving a pentacene precursor and a polystyrene in an organic solvent, the mobility is 0.05 cm²/Vs when the ratio of the pentacene precursor to the total of the pentacene precursor and the polymer is 99 mass%, but is reduced to 1/10, i.e., 0.005 cm²/Vs, when the ratio above is 50 mass%.

In Patent Document 6, in order to solve such a problem, a semiconductor solution is obtained by dissolving pentacene having a specific substituent in an organic solvent together with a polymer, and an organic semiconductor film is obtained using the obtained organic semiconductor solution.

In Patent Document 7, an organic semiconductor compound substituted with a substituent at a specific position, and a synthesis method thereof have been proposed.

### RELATED ART

### PATENT DOCUMENT

Patent Document 1: JP2008-290963
Patent Document 2: WO2008/50726
Patent Document 3: JP2009-81408
Patent Document 4: WO2011/024804
Patent Document 5: JP2005-505142
Patent Document 6: JP2007-519227
Patent Document 7: WO2013/021953

### SUMMARY OF THE INVENTION

### PROBLEMS TO BE SOLVED BY THE INVENTION

As described above, in Patent Document 6, it is demonstrated that by using, as an organic semiconductor compound, pentacene having a specific substituent, an organic semiconductor film having acceptable semiconductor characteristics is obtained even when the organic semiconductor compound is dissolved in an organic solvent together with a substantial amount of a polymer.

The present invention provides an organic semiconductor solution ensuring that even when such pentacene is not used as an organic semiconductor compound, the film formability is enhanced by virtue of containing a substantial amount of a polymer and an organic semiconductor film having acceptable semiconductor characteristics can be obtained.

### MEANS TO SOLVE THE PROBLEMS

The organic semiconductor solution of the present invention contains an organic solvent, and a polymer and an organic semiconductor precursor which are dissolved in the organic solvent, wherein the ratio of the polymer to the total of the polymer and the organic semiconductor precursor is 3 mass% or more, and the organic semiconductor precursor has a structure in which a dienophilic alkene is added in an eliminatable state through a double bond thereof to a benzene ring of an organic semiconductor compound of the following formula (I) :

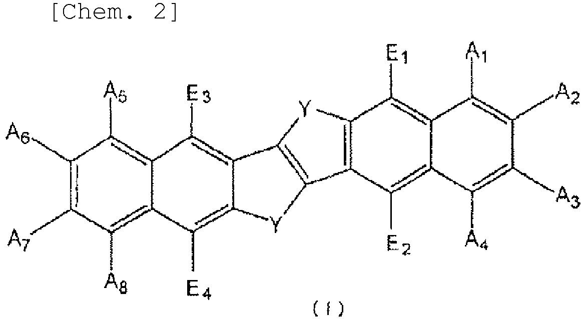

(wherein each of A₁ to A₈ and E₁ to E₄ is independently selected from the group consisting of hydrogen atom, halogen atoms, alkyl groups having from 1 to 20 carbon atoms, etc., and Y is sulfur or selenium).

In the organic semiconductor precursor, a dienophilic alkene is added "in an eliminatable state" through a double bond to a benzene ring of an organic semiconductor compound of formula (I), and this means that the dienophilic alkene can be eliminated and removed from the organic semiconductor precursor, for example, by pressure reduction and/or heating, without decomposing the organic semiconductor compound of formula (I).

The organic semiconductor film of the present invention is an organic semiconductor film formed of a polymer and an organic semiconductor compound,
wherein the ratio of the polymer to the total of the polymer and the organic semiconductor compound is 3 mass% or more, the organic semiconductor compound has the following formula (I), and the organic semiconductor film satisfies at least one of the following conditions (i) to (iii) : 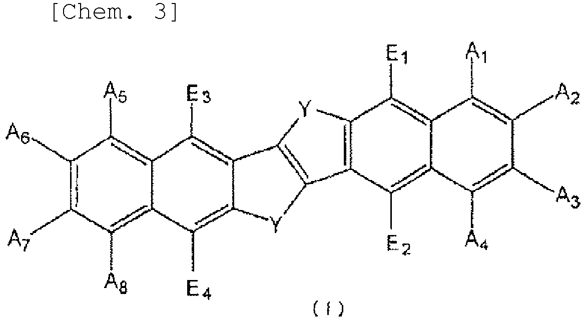 (wherein each of A₁ to A₈ and E₁ to E₄ is independently selected from the group consisting of hydrogen atom, halogen atoms, alkyl groups having from 1 to 20 carbon atoms, alkenyl groups having from 2 to 20 carbon atoms, alkynyl groups having from 2 to 20 carbon atoms, substituted or unsubstituted aromatic groups having from 4 to 20 carbon atoms, ketone groups having from 2 to 10 carbon atoms, amino groups having from 1 to 20 carbon atoms, amide groups having from 1 to 20 carbon atoms, imide groups having from 1 to 20 carbon atoms, sulfide groups having from 1 to 20 carbon atoms, and alkylsilylalkynyl groups having from 1 to 40 carbon atoms, two adjacent members of A₁ to A₈ may combine with each other to form a substituted or unsubstituted aromatic group having from 4 to 20 carbon atoms, and
Y is sulfur or selenium);
(i) the film has first and second layers stacked one on another, both of the first and second layers have the organic semiconductor compound, and the mass fraction of the organic semiconductor compound in the first layer is higher than the mass fraction of the organic semiconductor compound in the second layer,
(ii) the film further contains an organic semiconductor precursor, and the organic semiconductor precursor has a structure in which a dienophilic alkene is added in an eliminatable state through a double bond thereof to a benzene ring of the organic semiconductor compound of formula (I), and
(iii) the film contains a crystal of the organic semiconductor compound having a long axis diameter of more than 20 µm.

### EFFECTS OF THE INVENTION

According to the organic semiconductor solution of the present invention, the film formability is enhanced by virtue of containing a substantial amount of a polymer and an organic semiconductor film having acceptable semiconductor characteristics can be obtained. In addition, according to the organic semiconductor film of the present invention, excellent semiconductor characteristics can be provided.

### BRIEF DESCRIPTION OF THE DRAWINGS

[Fig. 1] Fig. 1 is a photograph when the green film produced in Example 1 is observed from the top surface.
[Fig. 2] Fig. 2 is a photograph when the organic semiconductor film produced in Example 1 is observed from the top surface by a polarizing microscope.
[Fig. 3] Fig. 3 is a photograph when the green film produced in Example 2 is observed from the top surface.
[Fig. 4] Fig. 4 is a photograph when the organic semiconductor film produced in Example 2 is observed from the top surface by a polarizing microscope.
[Fig. 5] Fig. 5 is a cross-sectional SEM photograph of the organic semiconductor film produced in Example 2.
[Fig. 6] Fig. 6 is an out-of-plane XRD observation result of the organic semiconductor film produced in Example 2.
[Fig. 7] Fig. 7 is an in-plane XRD observation result of the organic semiconductor film produced in Example 2.
[Fig. 8] Fig. 8 is a conceptual diagram of the organic semiconductor film produced in Example 2.
[Fig. 9] Fig. 9 is a diagram depicting the relationship between the mobility and the polymer content percentage in the organic semiconductor films produced in Examples 2 to 6.
[Fig. 10] Fig. 10 is a diagram depicting the transmission characteristics of the transistor obtained in Example 2.
[Fig. 11] Fig. 11 is a photograph when the green film produced in Comparative Example 2 is observed from the top surface.
[Fig. 12] Fig. 12 is a photograph when the organic semiconductor film produced in Example 7 is observed from the top surface by a polarizing microscope.
[Fig. 13] Fig. 13 is a diagram depicting the transmission characteristics of the transistor obtained in Example 7.
[Fig. 14] Fig. 14 is a photograph when the organic semiconductor film produced in Example 8 is observed from the top surface by a polarizing microscope.
[Fig. 15] Fig. 15 is a diagram depicting the transmission characteristics of the transistor obtained in Example 8.

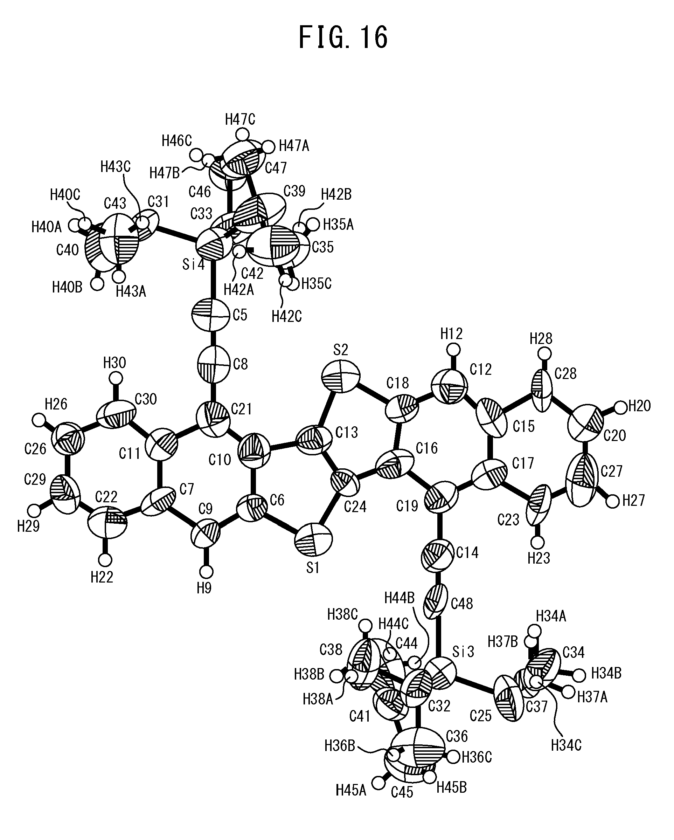

[Fig. 16] Fig. 16 is a molecular structure ORTEP drawing based on the single-crystal structural analysis of di-TIPS-substituted DNTT obtained in Reference Example 2.

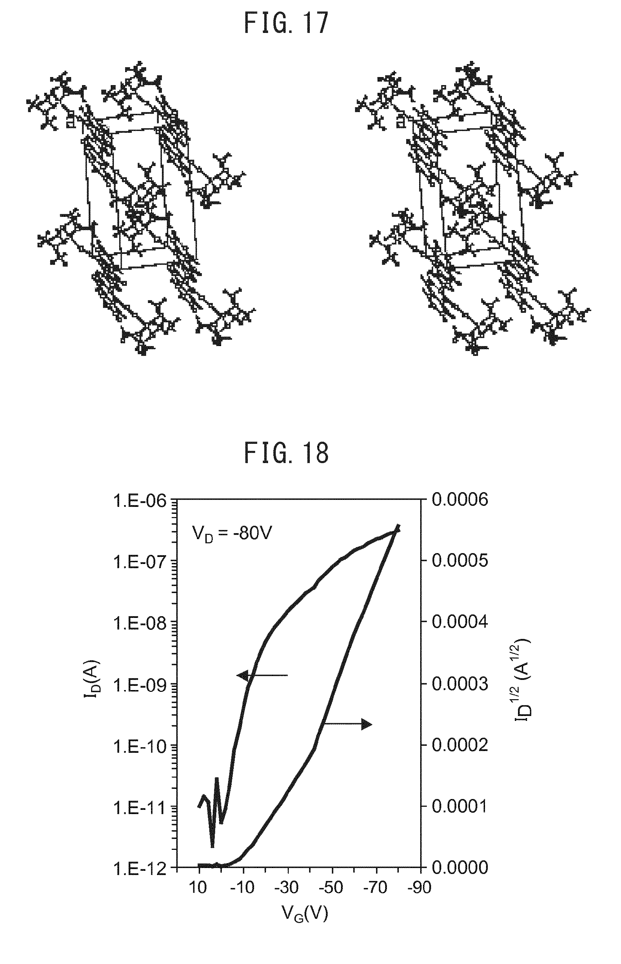

[Fig. 17] Fig. 17 is a crystal packing (stereo) diagram of di-TIPS-substituted DNTT obtained in Reference Example 2.
[Fig. 18] Fig. 18 is a diagram depicting the transmission characteristics of the transistor obtained in Reference Example 3.
[Fig. 19] Fig. 19 is a photograph when the organic semiconductor film produced in Example 9 is observed from the top surface by a polarizing microscope.
[Fig. 20] Fig. 20 is a diagram depicting the transmission characteristics of the transistor obtained in Example 9.
[Fig. 21] Fig. 21 is a photograph when the organic semiconductor film produced in Example 10 is observed from the top surface by a polarizing microscope.
[Fig. 22] Fig. 22 is a diagram depicting the transmission characteristics of the transistor obtained in Example 10.
[Fig. 23] Fig. 23 is a photograph when the organic semiconductor film produced in Example 11 is observed from the top surface by a polarizing microscope.
[Fig. 24] Fig. 24 is a diagram depicting the transmission characteristics of the transistor obtained in Example 11.
[Fig. 25] Fig. 25 is a photograph when the organic semiconductor film produced in Example 12 is observed from the top surface by a polarizing microscope.
[Fig. 26] Fig. 26 is a diagram depicting the transmission characteristics of the transistor obtained in Example 12.
[Fig. 27] Fig. 27 is a photograph when the organic semiconductor film produced in Example 13 is observed from the top surface by a polarizing microscope.
[Fig. 28] Fig. 28 is a diagram depicting the transmission characteristics of the transistor obtained in Example 13.

### MODE FOR CARRYING OUT THE INVENTION

### «Definition»

In the description of this specification, for the sake of simplifying the description, the expression "alkyl groups having from 1 to 20 carbon atoms, alkenyl groups having from 2 to 20 carbon atoms, alkynyl groups having from 2 to 20 carbon atoms, substituted or unsubstituted aromatic groups having from 4 to 20 carbon atoms, ketone groups having from 2 to 10 carbon atoms, amino groups having from 1 to 20 carbon atoms, amide groups having from 1 to 20 carbon atoms, imide groups having from 1 to 20 carbon atoms, sulfide groups having from 1 to 20 carbon atoms, and alkylsilylalkynyl groups having from 1 to 40 carbon atoms" is referred to as "alkyl groups having from 1 to 20 carbon atoms, etc.".

The "aromatic group" in the "substituted or unsubstituted aromatic group having from 4 to 20 carbon atoms" may be a benzene-based aromatic group, a heterocyclic group or a non-benzene-based aromatic group. Specific benzene-based aromatic groups include a benzene group and a naphthalene group. Specific heterocyclic groups include furan group, thiophene group, pyrrole group, and imidazole group. Specific non-benzene-based aromatic groups include annulene and azulene. In the case where such an aromatic group is substituted, the substituent includes alkyl groups, alkenyl groups, alkynyl groups, aromatic groups, ketone groups, amino groups, amide groups, imide groups, and sulfide groups.

For example, when the "substituted or unsubstituted aromatic group having from 4 to 20 carbon atoms" is a substituted or unsubstituted thiophene group, the thiophene group may be further substituted with a substituted or unsubstituted thiophene group, as shown below. 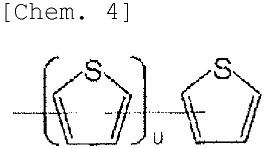 (wherein u is an integer of 0 to 4).

Incidentally, when the aromatic group such as thiophene group is substituted, the substituent may be halogen atoms, alkyl groups, alkenyl groups, alkynyl groups, substituted or unsubstituted aromatic groups, ketone groups, amino groups, amide groups, imide groups, sulfide groups, alkylsilylalkynyl groups, etc.

Also, the "alkylsilylalkynyl group having from 1 to 40 carbon atoms" may be a trialkylsilylalkynyl group having from 1 to 40 carbon atoms, particularly a trialkylsilylalkynyl group having the following formula:

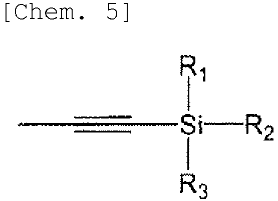

(wherein each of R₁ to R₃ is independently alkyl groups having from 1 to 10 carbon atoms, particularly a group selected from the group consisting of methyl group, ethyl group, isopropyl group, n-butyl group, sec-butyl group and tert-butyl group).

In the description of this specification, the "halogen" means fluorine, chlorine, bromine, iodine or astatine, particularly chlorine, bromine or iodine, more particularly bromine.

### <<Organic Semiconductor Solution>>

The organic semiconductor solution of the present invention contains an organic solvent, and a polymer and an organic semiconductor precursor which are dissolved in the organic solvent.

In the organic semiconductor solution of the present invention, the ratio of the polymer to the total of the polymer and the organic semiconductor precursor is 3 mass% or more, 5 mass% or more, 10 mass% or more, 20 mass% or more, or 25 mass% or more. The ratio may be, for example 90 mass% or less, 80 mass% or less, 70 mass% or less, 60 mass% or less, 50 mass% or less, or 45 mass% or less.

The organic semiconductor solution of the present invention may contain the organic semiconductor precursor at any concentration and, for example, may contain the organic semiconductor precursor at a concentration of 0.01 to 10 mass%, from 0.05 to 5 mass%, or from 0.1 to 3 mass%.

In the organic semiconductor solution of the present invention, the organic semiconductor precursor has a structure in which a dienophilic alkene is added in an eliminatable state through a double bond thereof to a benzene ring of an organic semiconductor compound of the following formula (I):

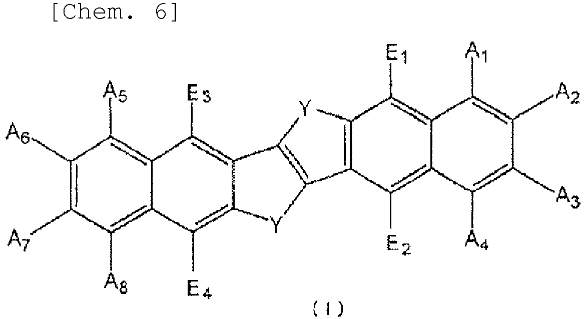

(wherein each of A₁ to A₈ and E₁ to E₄ is independently selected from the group consisting of hydrogen atom, halogen atoms, alkyl groups having from 1 to 20 carbon atoms, etc., two adjacent members of A₁ to A₈ may combine with each other to form a substituted or unsubstituted aromatic group having from 4 to 20 carbon atoms, and
Y is sulfur or selenium).

According to the organic semiconductor solution of the present invention, an organic semiconductor film formed of a polymer and an organic semiconductor compound of formula (I) can be obtained by coating the solution on a substrate to produce a green film, and applying light irradiation and/or heating to the green film to eliminate and remove the dienophilic alkene from the organic semiconductor precursor.

In this case, the organic semiconductor solution of the present invention can provide improved film formability by virtue of containing a relatively large amount of a polymer.

The organic semiconductor solution of the present invention can also provide a semiconductor film having unexpectedly good semiconductor characteristics, despite containing a relatively large amount of a polymer. In contrast, it has been heretofore considered that in the case of using an organic semiconductor solution containing a polymer and an organic semiconductor precursor as in Patent Document 5, when the polymer concentration is increased, the semiconductor characteristics of the obtained semiconductor film are significantly deteriorated.

Although the present invention is not limited by any theory, it is believed that, in the case of using a pentacene precursor as an organic semiconductor as in Patent Document 5 and increasing the polymer content, the semiconductor characteristics are significantly deteriorated for the following reason. That is, when using a general aromatic compound such as pentacene, a chemical interaction between molecules causing crystallization is suppressed by inhibition of a large amount of a polymer component, and in turn, crystal growth hardly proceeds in a continuous layer which is advantageous for expressing semiconductor characteristics.

On the other hand, in the case of using an organic semiconductor compound of formula (I), i.e., a condensed polycyclic aromatic compound having a thienothiophene moiety, as in the present invention, it is believed that the intermolecular interaction causing crystallization of the compound is very strong and when decomposing the organic semiconductor precursor to obtain the organic semiconductor compound of formula (I), the polymer and the organic semiconductor compound of formula (I) are separated, allowing organic semiconductor compounds of formula (I) to aggregate, as a result, crystallization of the semiconductor compound of formula (I) is promoted without being inhibited by the polymer. In addition, the skeleton moiety of the organic semiconductor compound of formula (I) has high aromaticity, which is believed to also strongly promote the crystallization of the organic semiconductor compound of formula (I).

Furthermore, in the case of using the organic semiconductor compound of formula (I) in combination with an appropriate amount of a polymer as in the present invention, it is believed that the diffusion rate of the organic semiconductor compound in the polymer is suppressed by the polymer and the crystallization rate is appropriately controlled, as a result, an organic semiconductor film having a relatively large crystal is obtained.

### <<Organic Semiconductor Solution - Polymer>>

The polymer used in the organic semiconductor solution of the present invention may be any polymer that can be dissolved in the organic solvent used.

As the polymer for use in the present invention, a polymer in which the repeating unit of the polymer has a conjugated double bond and/or an aromatic ring, may be selected. In addition, as the polymer used, a polymer in which the repeating unit of the polymer does not contain an element other than carbon, hydrogen and halogens, particularly a polymer in which the repeating unit of the polymer does not contain an element other than carbon and hydrogen, may be selected.

Such a polymer is thought to be preferred so as to prevent the polymer from inhibiting crystallization of the organic semiconductor compound of formula (I) and/or semiconductor characteristics.

Furthermore, as the polymer used, an amorphous polymer selected from the group consisting of polycarbonate, polystyrene, acrylic resin, methacrylic resin, polyvinyl chloride, polyphenylene ether and polysulfone may be selected. In particular, as the amorphous polymer, a styrene-based polymer having a benzene ring moiety in the repeating unit, a polycarbonate-based polymer having a benzene ring moiety and a carbonate group in the repeating unit, an acrylic polymer composed of a polymer of an acrylic acid ester or a methacrylic acid ester, a polysilane-based polymer, or a combination thereof may be selected.

Such an amorphous polymer is considered to keep the amorphous property when separating the polymer and the organic semiconductor compound of formula (I) and crystallizing the organic semiconductor compound and at the same time, allow for slow progress of thermal diffusion of the organic semiconductor compound that has a low molecular weight, thereby promoting the separation of the polymer and the organic semiconductor compound and improving the crystallinity of the organic semiconductor compound.

The polymer used may be an insulating polymer or a semiconducting polymer.

Examples of the insulating polymer include a polycarbonate, a polyvinyl, a polyimide, a polyether, and a polyester. Specifically, the polyvinyl includes polyethylene, polypropylene, butadiene, polystyrene, poly(α-methylstyrene), polyvinyl acetate, and polyvinylpyrrolidone. The polyether includes a polyphenylene ether such as poly(2,6-dimethyl-1,4-phenylene oxide. The polyester includes polymethyl methacrylate, polyethylene terephthalate, and polyethylene glycol dimethacrylate. The insulating polymer also includes acetyl cellulose, particularly triacetyl cellulose.

Examples of the semiconductor polymer includes polythienylene-vinylene, polyaniline, polypyrrole, and polyfuranylene-vinylene.

The polymer for use in the present invention may have a molecular weight of, for example, 1,000 or more, 3,000 or more, 5,000 or more, or 10,000 or more. In addition, the polymer used may be thermoplastic and at the same time, may have a glass transition temperature of 100°C or more, 120°C or more, 150°C or more, or 200°C or more.

### <<Organic Semiconductor Solution - Organic Solvent>>

The organic solvent used in the organic semiconductor solution of the present invention may be any organic solvent capable of dissolving the polymer and organic semiconductor precursor used.

Accordingly, for example, an aprotic polar solvent such as N-methylpyrrolidone, dimethylsulfoxide, acetonitrile and ethyl acetate; an ether-based solvent such as diethyl ether, tetrahydrofuran, diisopropyl ether, diethylene glycol dimethyl ether and 1,4-dioxane; aromatic hydrocarbons such as benzene, toluene, xylene and mesitylene (i.e., 1,3,5-trimethylbenzene); aliphatic hydrocarbons such as hexane and heptane; and a halogen-containing solvent such as dichloromethane, chloroform and dichloroethane may be considered as the usable solvent.

### <<Organic Semiconductor Solution - Organic Semiconductor Precursor>>

The organic semiconductor precursor used in the organic semiconductor solution of the present invention is an organic semiconductor precursor having a structure in which a dienophilic alkene is added in an eliminatable state through a double bond thereof to a benzene ring of an organic semiconductor compound of formula (I), and may be any organic semiconductor precursor that can be dissolved in the organic solvent used.

Accordingly, the organic semiconductor precursor may have, for example, a structure of the following formula (I-1) :

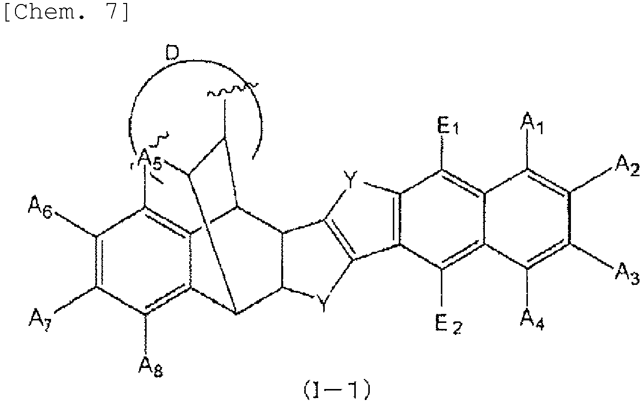

(wherein each of A₁ to A₈, E₁ and E₂ is independently selected from the group consisting of hydrogen atom, halogen atoms, alkyl groups having from 1 to 20 carbon atoms, etc., two adjacent members of A₁ to A₈ may combine with each other to form a substituted or unsubstituted aromatic group having from 4 to 20 carbon atoms,
D is a substituent obtained by adding a dienophilic alkene to a benzene ring, and
Y is sulfur or selenium).

The organic semiconductor precursor may have particularly a structure of the following formula (1-1-1) :

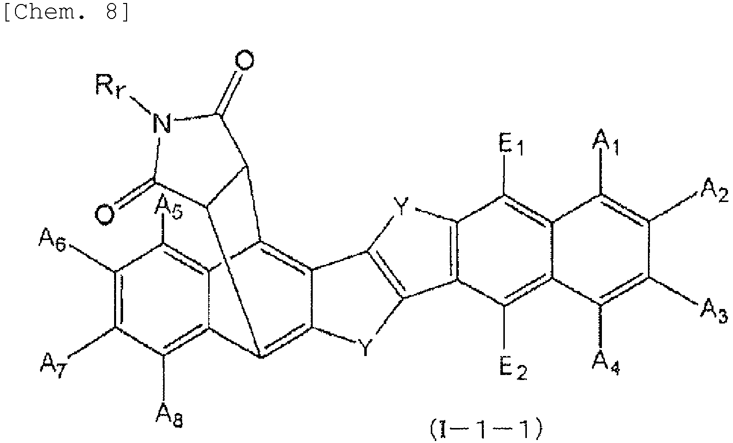

(wherein Rᵣ is as described below).

With regard to specific organic semiconductors, organic semiconductor compounds and dienophilic alkenes, Patent Document 4, etc. may be referred to.

With regard to the synthesis method of the organic semiconductor compound of formula (I) where E₁ to E₄ are substituted, the following description and Reference Examples in the description of this specification may be referred to.

### <Synthesis Method 1 of Organic Semiconductor Compound of Formula (I) where E₁ to E₄ are substituted>

The organic semiconductor compound of formula (I) where at least a part of E₁ to E₄ are substituted with a halogen atom, can be synthesized by a method including the following steps:
(a) providing a composition containing an organic solvent and a condensed polycyclic aromatic compound of the following formula (I'): 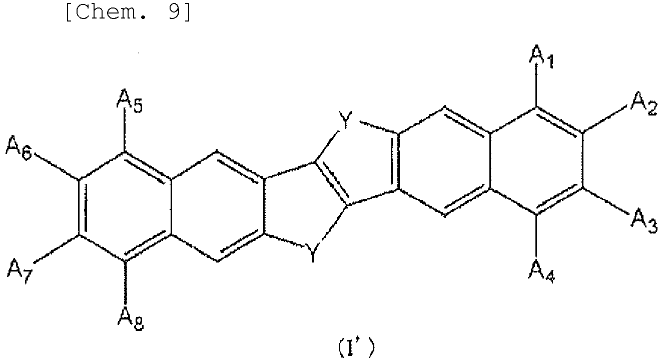 (wherein each of A₁ to A₈ is independently selected from the group consisting of hydrogen atom, halogen atoms, alkyl groups having from 1 to 20 carbon atoms, etc., two adjacent members of A₁ to A₈ may combine with each other to form a substituted or unsubstituted aromatic group having from 4 to 20 carbon atoms, and
   Y is sulfur or selenium); and
(b) adding a halogen to the composition above.

The organic solvent that can be used here includes any organic solvent capable of dissolving and/or dispersing the condensed polycyclic aromatic compound of formula (I'). Specifically, as this organic solvent, the organic solvents recited with regard to the organic semiconductor solution of the present invention may be considered.

Addition of a halogen to the composition containing the condensed polycyclic aromatic compound of formula (I') can be performed by any method. For example, fluorine or chlorine can be added by bubbling, and bromine, iodine or astatine can be added as a liquid or a solid. In order to promote the reaction of the compound of formula (I') and a halogen, heating may be performed.

<Synthesis Method 2 of Organic Semiconductor Compound of Formula (I) where E₁ to E₄ are substituted>

The organic semiconductor compound of formula (I) where at least a part of E₁ to E₄ are substituted with alkyl groups having from 1 to 20 carbon atoms, etc., can be synthesized by a method including the following steps:
(a) providing a composition containing an organic solvent and an organic semiconductor compound of formula (I) where at least a part of E₁ to E₄ are substituted with a halogen atom; and
(b) substituting a halogen atom of the organic semiconductor compound of formula (I) by a substituent selected from the group consisting of alkyl groups having from 1 to 20 carbon atoms, etc.

The organic solvent that can be used here includes any organic solvent capable of dissolving and/or dispersing the organic semiconductor compound of formula (I) where at least a part of E₁ to E₄ are substituted with a halogen atom. Specifically, as this organic solvent, the organic solvents recited with regard to the organic semiconductor solution of the present invention may be considered.

The reaction of substituting a halogen atom of the organic semiconductor compound of formula (I) by a substituent selected from the group consisting of alkyl groups having from 1 to 20 carbon atoms, etc. can be performed by various coupling methods using an aromatic halide, i.e. Mizoroki-Heck reaction, Negishi coupling, Migita-Kosugi-Stille coupling, Sonogashira coupling, Suzuki-Miyaura coupling, Buchwald-Hartwig reaction or Kumada-Tamao-Corriu coupling. In order to promote this coupling reaction, heating may be performed.

The outline of each coupling reaction is as follows (Ar is an aromatic moiety, X is a halogen, and R is hydrogen, alkyl groups having from 1 to 20 carbon atoms, etc.):
(1) Mizoroki-Heck Reaction

   Ar-X + H₂C=CHR (+ Pd catalyst) → Ar-HC=CHR
(2) Negishi Coupling

   Ar-X + R-Zn-X (+ Pd catalyst) → Ar-R
(3) Migita-Kosugi-Stille Coupling

   Ar-X + R-Sn-R'₃ (+ Pd catalyst) → Ar-R
(4) Sonogashira Coupling

   Ar-X + R-C=C-H (+ Pd catalyst) + base → Ar-C=C-R
(5) Suzuki-Miyaura Coupling

   Ar-X + R-B (OH)₂ (+ Pd catalyst) + base → Ar-R
(6) Buchwald-Hartwig Reaction

   Ar-X + R-NH₂ (+ Pd catalyst) + base→ Ar-NHR
(7) Kumada-Tamao-Corriu Coupling

   Ar-X + R-Mg-X (+ Ni catalyst) → Ar-R

### <<Organic Semiconductor Solution - Organic Semiconductor Precursor - Dienophilic Alkene>>

In the organic semiconductor precursor used in the organic semiconductor solution of the present invention, the dienophilic alkene may be any compound capable of being added in an eliminatable state to a benzene ring of the organic semiconductor compound of formula (I).

With regard to the dienophilic alkene itself and the method for adding such a dienophilic alkene to a benzene ring of an organic semiconductor compound of formula (I), Patent Document 4 may be referred to.

Specifically, the dienophilic alkene includes, for example, compounds of the following formulae (II-1a) and (II-2a), particularly the following formulae (II-1b) and (II-2b), more particularly the following formulae (II-1c) and (II-2c):

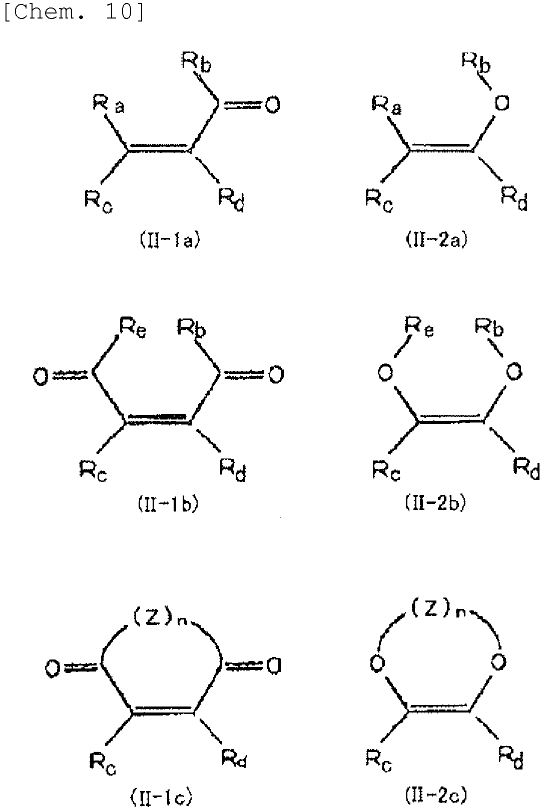

(wherein each of Rₐ, R_{b}, R_{c} and R_{d} is independently selected from the group consisting of a bond, hydrogen, halogens, hydroxyl group, amide groups, mercapto group, cyano group, alkyl groups having from 1 to 10 carbon atoms, alkenyl groups having from 2 to 10 carbon atoms, alkynyl groups having from 2 to 10 carbon atoms, alkoxy groups having from 1 to 10 carbon atoms, substituted or unsubstituted aromatic groups having from 4 to 10 carbon atoms, ester groups having from 1 to 10 carbon atoms, ether groups having from 1 to 10 carbon atoms, ketone groups having from 1 to 10 carbon atoms, amino groups having from 1 to 10 carbon atoms, amide groups having from 1 to 10 carbon atoms, imide groups having from 1 to 10 carbon atoms, and sulfide groups having from 1 to 10 carbon atoms,
Rₐ and R_{b} may combine with each other to form a ring,
R_{c} and R_{d} may combine with each other to form a ring,
n is an integer of 1 to 5, and
Z is selected from the group consisting of a bond (-), oxygen (-O-), methylenic carbon (-C(Rr)₂-), an ethylenic carbon (-C(Rᵣ)=), carbonyl group (-C(=O)-), nitrogen (-N(Rᵣ)-) and sulfur (-S-), and when n is 2 or more, each may be the same as or different from every other (each Rᵣ is independently selected from the group consisting of hydrogen, halogens, alkyl groups having from 1 to 10 carbon atoms, alkenyl groups having from 2 to 10 carbon atoms, alkynyl groups having from 2 to 10 carbon atoms, alkoxy groups having from 1 to 10 carbon atoms, substituted or unsubstituted aromatic groups having from 4 to 10 carbon atoms, ester groups having from 1 to 10 carbon atoms, ether groups having from 1 to 10 carbon atoms, ketone groups having from 1 to 10 carbon atoms, amino groups having from 1 to 10 carbon atoms, amide groups having from 1 to 10 carbon atoms, imide groups having from 1 to 10 carbon atoms, and sulfide groups having from 1 to 10 carbon atoms).

More specifically, the dienophilic alkene includes compounds of the following formulae (II-1-1) to (II-2-3):

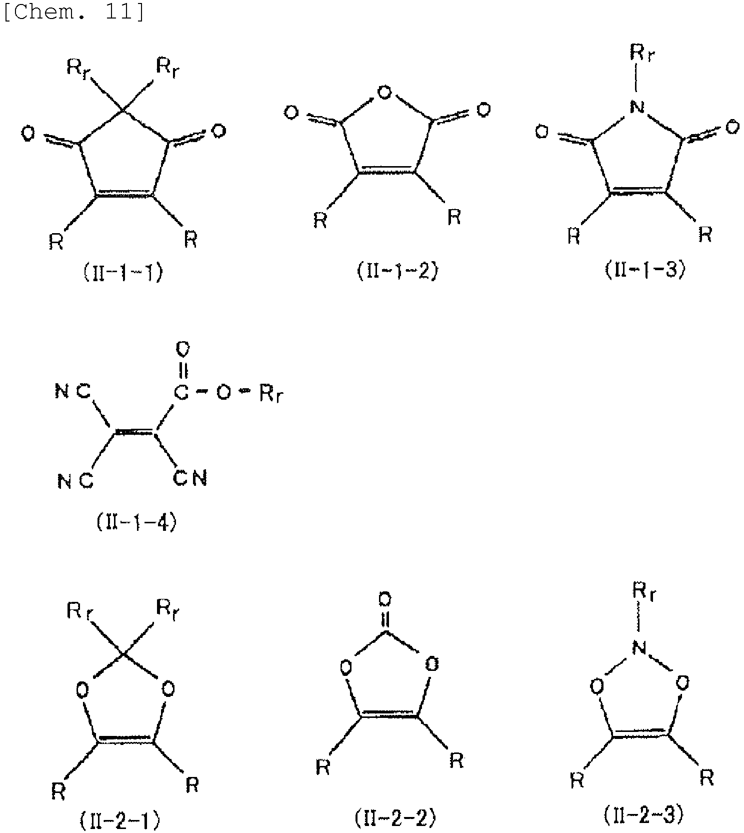

(wherein each of R and Rᵣ is independently selected from the group consisting of hydrogen, halogens, hydroxyl group, amide groups, mercapto group, cyano group, alkyl groups having from 1 to 10 carbon atoms, alkenyl groups having from 2 to 10 carbon atoms, alkynyl groups having from 2 to 10 carbon atoms, alkoxy groups having from 1 to 10 carbon atoms, substituted or unsubstituted aromatic groups having from 4 to 10 carbon atoms, ester groups having from 1 to 10 carbon atoms, ether groups having from 1 to 10 carbon atoms, ketone groups having from 1 to 10 carbon atoms, amino groups having from 1 to 10 carbon atoms, amide groups having from 1 to 10 carbon atoms, imide groups having from 1 to 10 carbon atoms, and sulfide groups having from 1 to 10 carbon atoms).

### «Production Method of Organic Semiconductor Film»

The method of the present invention for producing an organic semiconductor film includes coating the organic semiconductor solution of the present invention on a substrate to produce a green film, and applying light irradiation and/or heating to the green film to eliminate and remove the dienophilic alkene from the precursor and obtain an organic semiconductor film formed of a polymer and an organic semiconductor compound of formula (I).

Coating of the solution on a substrate may be performed in any manner and, for example, may be performed by a casting method, a spin coating method or a printing method, particularly by a casting method such as drop casting method.

In the case of eliminating and removing the dienophilic alkene by light irradiation and/or heating, arbitrary conditions involving substantially no decomposition of the organic semiconductor compound of formula (I) may be employed. Accordingly, elimination and removal of the dienophilic alkene may be performed by heating, for example, at a temperature of 80°C or more, 100°C or more, 120°C or more, or 140°C or more, and 200°C or less, 220°C or less, 240°C or less, or 260°C or less.

In addition, elimination and removal of the dienophilic alkene may be performed, for example, under reduced pressure or atmospheric pressure. Furthermore, elimination and removal of the dienophilic alkene may be performed, for example, in a nitrogen atmosphere or an air atmosphere. In particular, elimination and removal of the dienophilic alkene are preferably performed in an air atmosphere under atmospheric pressure, because the production of an organic semiconductor film is facilitated.

With regard to the elimination and removal of the dienophilic alkene, Patent Document 4 may be referred to.

### <<Manufacturing Method of Organic Semiconductor Device>>

The method of the present invention for manufacturing an organic semiconductor device includes producing an organic semiconductor film by the method of the present invention for producing an organic semiconductor film. This method may optionally further include forming an electrode layer and/or a dielectric layer on the top side or bottom side of the organic semiconductor film.

### <<Organic Semiconductor Film>>

The organic semiconductor film of the present invention is formed of a polymer and an organic semiconductor compound, wherein the ratio of the polymer to the total of the polymer and the organic semiconductor compound is 3 mass% or more, the organic semiconductor compound has the following formula (I), and the organic semiconductor film satisfies at least one of the following conditions (i) to (iii).

### <<Organic Semiconductor Film - Condition (i)>>

The condition (i) of the organic semiconductor film of the present invention is that the organic semiconductor film has first and second layers stacked one on another, both of the first and second layers have the organic semiconductor compound, and the mass fraction of the organic semiconductor compound in the first layer is higher than the mass fraction of the organic semiconductor compound in the second layer.

In this organic semiconductor film of the present invention, the mass fraction of the organic semiconductor compound in the first layer may be 1.1 times or more, 1.2 times or more, 1.3 times or more, 1.4 times or more, 1.5 times or more, 1.6 times or more, 1.7 times or more, 1.8 times or more, 1.9 times or more, or 2.0 times or more, the mass fraction of the semiconductor compound in the second layer.

In such an organic semiconductor device of the present invention, crystallization of the organic semiconductor compound in the first layer is promoted by virtue of a relatively high content percentage of the organic semiconductor compound in the first layer, so that high semiconductor characteristics can be provided.

### <<Organic Semiconductor Film - Condition (ii)>>

The condition (ii) of the organic semiconductor film of the present invention is that the organic semiconductor film further contains an organic semiconductor precursor and the organic semiconductor precursor has a structure in which a dienophilic alkene is added in an eliminatable state through a double bond thereof to a benzene ring of an organic semiconductor compound of formula (I).

Containing an organic semiconductor precursor in the organic semiconductor film means that the organic semiconductor film contains a detectable amount of an organic semiconductor precursor. Accordingly, for example, the molar ratio of the organic semiconductor precursor may be more than 1 ppm, more than 10 ppm, more than 100 ppm, more than 1,000 ppm, or more than 10,000 ppm (1%), based on the semiconductor compound. Also, the ratio of the organic semiconductor precursor may be 10 mol% or less, 5 mol% or less, 3 mol% or less, 1 mol% or less, 0.1 mol% or less, or 0.01 mol% or less.

Such an organic semiconductor film of the present invention can have characteristics as an organic semiconductor film, despite containing an organic semiconductor precursor together with an organic semiconductor compound of formula (I). More specifically, in the case of producing the organic semiconductor film of the present invention by the method of the present invention, even when thermal elimination of the dienophilic alkene from the organic semiconductor precursor does not proceed completely, the organic semiconductor film of the present invention can have characteristics as a semiconductor film. This facilitates the production of the organic semiconductor film of the present invention and is preferred.

### <<Organic Semiconductor Film - Condition (iii)>>

The condition (iii) of the organic semiconductor film of the present invention is that the organic semiconductor film contains a crystal of the organic semiconductor compound having a long axis diameter of more than 20 µm, more than 30 µm, more than 40 µm, more than 50 µm, more than 60 µm, more than 70 µm, more than 80 µm, more than 90 µm, or more than 100 µm.

In such an organic semiconductor film of the present invention, the semiconductor characteristics, for example, the carrier mobility and the on/off ratio, can be improved by virtue of having a large crystal.

### <<Organic Semiconductor Film - Others>>

In the organic semiconductor film of the present invention, the ratio of the polymer to the total of the polymer, the organic semiconductor compound and the optional organic semiconductor precursor may be 5 mass% or more, 10 mass% or more, or 15 mass% or more. Also, the ratio may be 90 mass% or less, 80 mass% or less, 70 mass% or less, 60 mass% or less, 50 mass% or less, 40 mass% or less, 35 mass% or less, 30 mass% or less, or 25 mass% or less.

The organic semiconductor film of the present invention preferably has a diffraction peak in the in-plane XRD observation, particularly has a diffraction peak at 2θ of around 18°, around 23° and around 27°, and at the same time, has a diffraction peak at 2θ of around 5.5° in the out-of-plane XRD observation.

In such an organic semiconductor film of the present invention, the organic semiconductor compound constituting the organic semiconductor film can have characteristics comparable to an organic semiconductor film obtained by a vapor deposition method.

These organic semiconductor films of the present invention can be obtained by the method of the present invention for producing an organic semiconductor film.

### <<Organic Semiconductor Device>>

The organic semiconductor device is an organic semiconductor device having the organic semiconductor film of the present invention.

In particular, the organic semiconductor device of the present invention is a thin-film transistor having a source electrode, a drain electrode, a gate electrode, a gate insulating film and the organic semiconductor film of the present invention, and this is a thin-film transistor in which the source electrode and the drain electrode are insulated from the gate electrode by the gate insulating film and the current flowing through the organic semiconductor from the source electrode to the drain electrode is controlled by the voltage applied to the gate electrode.

Also, in particular, the organic semiconductor device of the present invention is a solar cell having the organic semiconductor film as an active layer.

Incidentally, the "organic semiconductor device" as used in the present invention means a device having an organic semiconductor film, and other layers such as electrode layer and dielectric layer may be formed of an inorganic material or an organic material.

### EXAMPLES

### «Example 1»

### <Preparation of Organic Semiconductor Precursor>

[1]Benzothieno[3,2-b][1]benzothiophene (hereinafter referred to as "DNTT") (structural formula shown below, molecular weight: 340.46) as an organic semiconductor compound was synthesized by the method described in Patent Document 1.

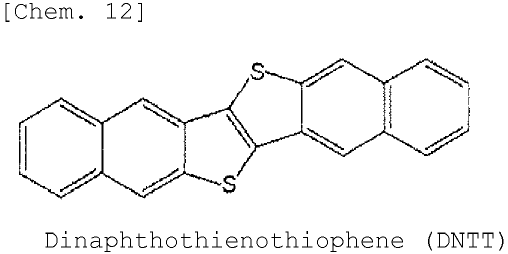

N-Phenylmaleimide (PMI, structural formula shown below, molecular weight: 173.16) was added to the obtained DNTT as in Example 1-10 of Patent Document 4 to obtain a DNTT precursor as an organic semiconductor precursor.

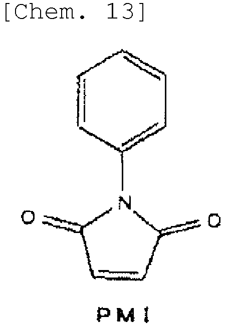

The obtained DNTT precursor had the following formula:

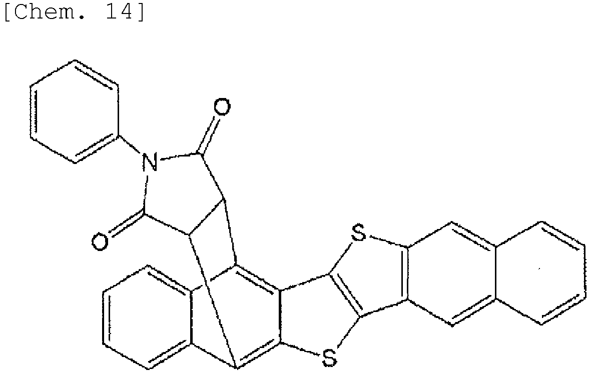

### <Preparation of Organic Semiconductor Solution>

An organic semiconductor solution was prepared by dissolving the DNTT precursor and a polystyrene (Ardrich Chemical, mass average molecular weight: 280,000 (GPC)) in a chloroform solution such that the mass ratio between DNTT precursor and polystyrene becomes 7:3 (DNTT precursor : polystyrene), i.e., such that the ratio of the polymer to the total of the polymer and the organic semiconductor precursor becomes 30 mass%. Here, the concentration of the DNTT precursor in the organic semiconductor solution was 0.2 mass%.

### <Formation of Organic Semiconductor Film>

An n-doped silicon wafer (surface resistance: 0.005 Ω·cm) having an SiO₂ oxide film of 300 nm was used as the substrate. Onto the SiO₂ oxide film of this substrate, the organic semiconductor solution was applied by drop casting to form a green film containing polystyrene and DNTT precursor.

### <Evaluation of Organic Semiconductor Film - Thickness Uniformity>

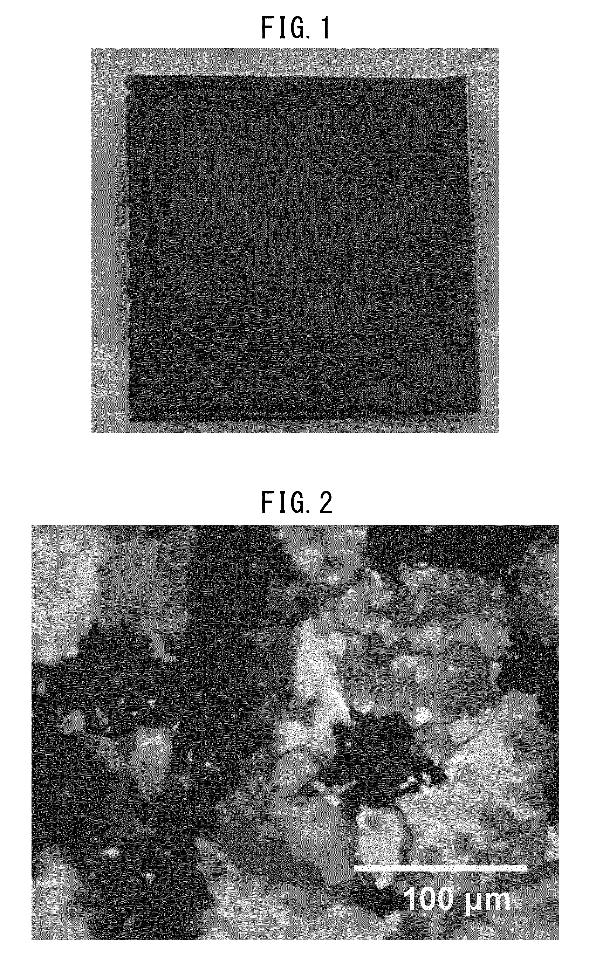

Fig. 1 shows a photograph when the green film formed is observed from the top surface. This film had a thickness of 300 nm and a uniform in-plane thickness (maximum difference in height: 20 nm or less).

### <Evaluation of Organic Semiconductor Film - Crystal Grain Size>

The green film was heated over 1 minute under the atmosphere on a hot plate heated at 250°C to eliminate PMI from the DNTT precursor and convert the precursor into DNTT, and then allowed to cool to form an organic semiconductor film. Fig. 2 shows a photograph when the organic semiconductor film obtained is observed from the top surface by a polarizing microscope. It was confirmed from Fig. 2 that the organic semiconductor film has a large crystal grain with a grain size of more than 100 µm.

### <Evaluation of Organic Semiconductor Film - Semiconductor Characteristics>

In the region of 1.4 cm×1.4 cm of this organic semiconductor film, source/drain gold electrodes having a channel width of 50 µm and a channel length of 1.5 mm were formed by a vapor deposition method. In this way, 16 bottom-gate top-contact field-effect transistors were fabricated.

All of 16 transistors obtained above exhibited a good mobility of 0.1 cm²/Vs or more and a good on-off current ratio of 10⁵ or more.

### «Comparative Example 1»

In Comparative Example 1, an organic semiconductor film was formed and evaluated in the same manner as in Example 1 except that polystyrene was not added to the organic semiconductor solution.

The film obtained by drop casting had concentrically extending wave-like unevenness. The thickness of this film had a maximum difference in height of more than 100 nm.

In addition, the semiconductor characteristics were evaluated by the same method as in Example 1, as a result, half of 16 devices exhibited a mobility of less than 0.1 cm²/Vs.

### <<Example 2>>

### <Preparation of Organic Semiconductor Solution>

An organic semiconductor solution was prepared in the same manner as in Example 1 except that the ratio of the polymer to the total of the polymer and the DNTT precursor was changed to 33 mass%. More specifically, the ratio of the polymer to the total of the polymer and the DNTT precursor was 33 mass%, chloroform was used as the solvent, and the concentration of the DNTT precursor in the organic semiconductor solution was 0.2 mass%.

### <Formation of Green Film>

An n-doped silicon wafer (surface resistance: 0.005 Ω·cm) having an SiO₂ oxide film of 300 nm was used as the substrate, and the surface of the silicon wafer was subjected to a chemical hydrophobic treatment (OTS treatment) with octadecyltrichlorosilane (OTS, LS-6495, Shin-Etsu Silicone). The water contact angle of the substrate after the hydrophobic treatment was 108°. Onto the OTS-treated SiO₂ oxide film of the substrate, the organic semiconductor solution was applied by drop casting to form a green film containing polystyrene and DNTT precursor.

### <Evaluation of Green Film - Thickness Uniformity>

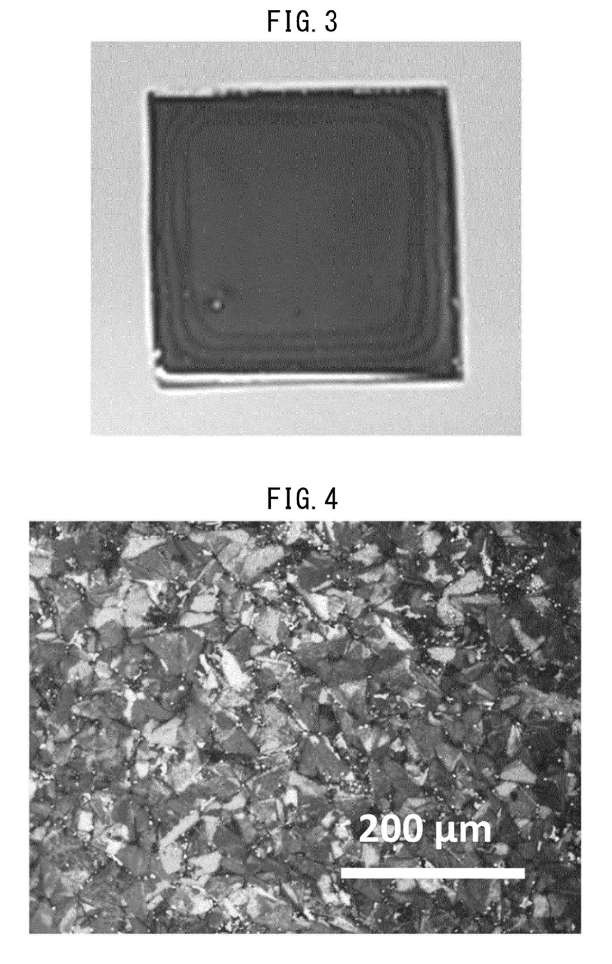

Fig. 3 shows a photograph when the formed green film is observed from the top surface. This film had a thickness of 300 nm and a uniform in-plane thickness (maximum difference in height: 20 nm or less).

### <Evaluation of Organic Semiconductor Film - Crystal Grain Size>

The green film was heated over 10 minutes under the atmosphere on a hot plate heated at 200°C to eliminate PMI from the DNTT precursor and convert the precursor into DNTT, and then allowed to cool to form an organic semiconductor film. Fig. 4 shows a photograph when the organic semiconductor film obtained is observed from the top surface by a polarizing microscope. It was confirmed from Fig. 4 that the organic semiconductor film has a large crystal grain with a grain size of more than 10 µm.

### <Evaluation of Organic Semiconductor Film - SEM, TEM and EELS Observations>

In addition, the organic semiconductor film was analyzed using a scanning electron microscope (SEM, Hitachi High-Technologies S-5200) and X-ray diffraction (XRD, Rigaku R-AXIS RAPID-S).

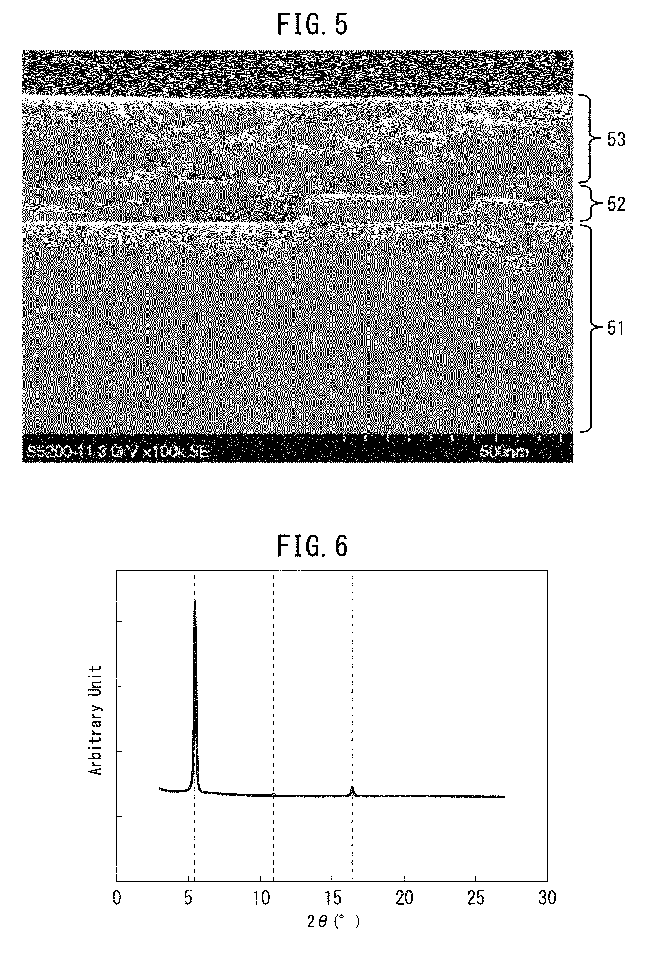

Fig. 5 shows the results of the cross-sectional SEM observation. It is understood from this figure that in the organic semiconductor film formed from an organic semiconductor solution containing a polymer and a DNTT precursor, a two-layer structure having a lower layer 52 and an upper layer 53 on a substrate 51 is obtained.

Furthermore, according to EELS (electron energy-loss spectroscopy) observation of the lower layer 52 and the upper layer 53), the constituent elements of each layer were as shown in Table 1 below. In Table 1, the value in the parenthesis is the value assuming that the total number of atoms of carbon (C) and sulfur (S) is 100 atm%.

[Table 1]

**Tale 1**

| Element | Lower Layer 52 (atm%) | Upper Layer 53 (atm%) | |
|---|---|---|---|
| | | Central Portion | Outer Portion |
| C | 89.8 (91.9) | 94.1 (96.3) | 97.4 (99.8) |
| O | 1.6 (-) | 1.7 (-) | 1.8 (-) |
| Al | 0.2 (-) | 0.2 (-) | 0.3 (-) |
| Si | 0.5 (-) | 0.4 (-) | 0.4 (-) |
| S | 7.9 (8.1) | 3.6 (3.7) | 0.2 (0.2) |

Since the atomic composition of DNTT ([1]benzothieno[3,2-b][1]benzothiophene) used as the organic semiconductor compound is C₂₂H₁₂S₂, the layer composed of absolutely only DNTT has an atomic composition where assuming that the total number of atoms of carbon (C) and sulfur (S) is 100 atm%, theoretically, carbon (C) is 91.7 atm% and sulfur (S) is 8.3 atm%.

On the other hand, the polystyrene used as the polymer is composed of only carbon (C) and hydrogen (H), and therefore the layer composed of absolutely only polystyrene comes to have an atomic composition where in the elemental analysis above, theoretically, carbon (C) is 100 atm%.

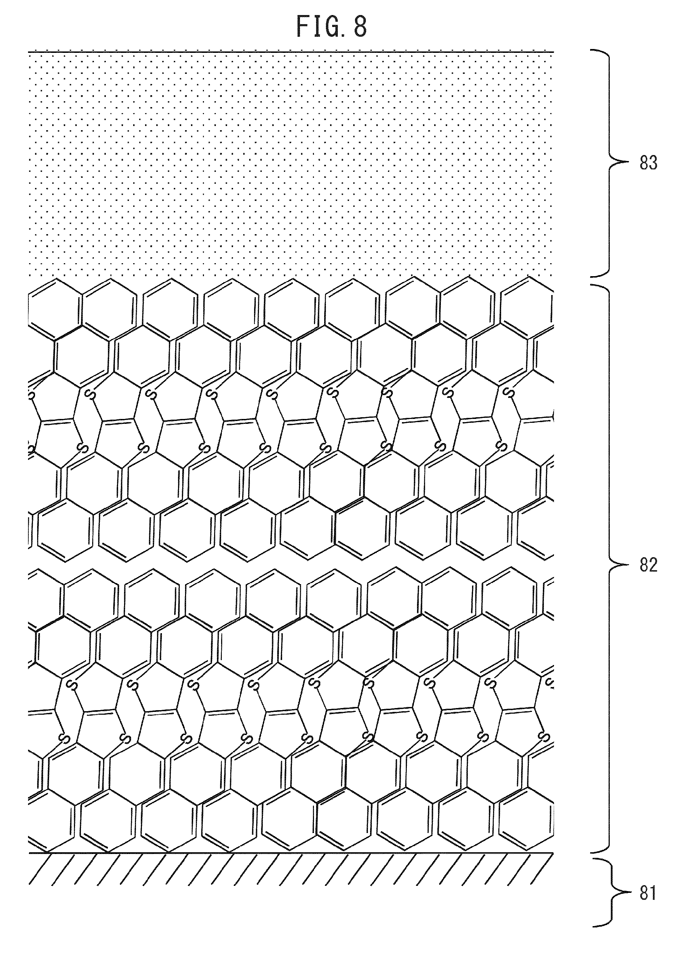

This way of thinking leads to the recognition that all of sulfur atoms present in the lower and upper layers are derived from DNTT. Accordingly, it is understood from Table 1 that in the organic semiconductor film obtained in this Example, as in the conceptual diagram illustrated in Fig. 8, a lower layer 82 mainly composed of DNTT is stacked on a substrate 81 and an upper layer 83 mainly composed of polymer is stacked thereon. More specifically, it is seen from Table 1 that in the organic semiconductor film obtained in this Example, the content percentage of DNTT in the lower layer 82 is more than 2 times (8.1 atm%/3.6 atm% = 2.25) the content percentage of DNTT in the central portion of the upper layer 83.

### <Evaluation of Organic Semiconductor Film - XRD Observation>

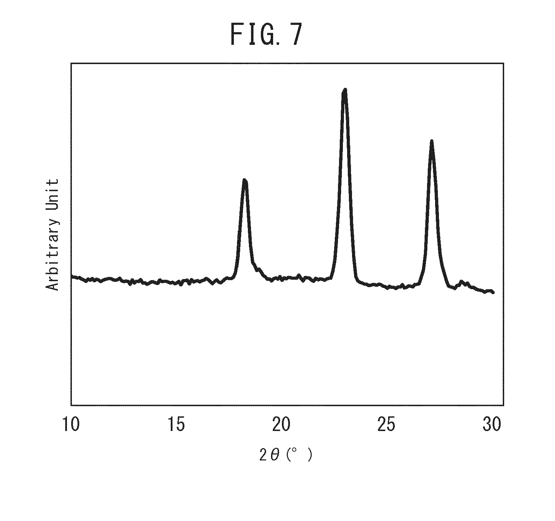

Fig. 6 shows the result of XRD observation performed out-of-plane, and Fig. 7 shows the result of XRD observation performed in-plane. It is understood from Fig. 6 and 7 that the organic semiconductor film obtained has a clear diffraction peak in both the out-of-plane and in-plane directions, i.e., has high crystallinity. More specifically, the organic semiconductor film has a diffraction peak in the in-plane XRD observation result (Fig. 7), particularly, has a diffraction peak at 2θ of around 18°, around 23° and around 27°, leading to an understanding that the organic semiconductor film is a polycrystal. In addition, since the organic semiconductor film has, in the out-of-plane XRD observation result (Fig. 6), a diffraction peak at 2θ of around 5.5°, i.e., has a diffraction peak at the position corresponding to a plane-to-plane spacing of about 15.5 Å coming under the length in the longitudinal direction of a DNTT molecule, it is understood that DNTT is oriented to stand erect from the substrate (edge-on orientation).

Accordingly, from the results of XRD observation performed out-of-plane and in-plane, it is understood that in the organic semiconductor film obtained in this Example, a lower layer 82 composed of DNTT is stacked on a substrate 81 and an upper layer 83 composed of polymer is stacked thereon, as in the conceptual diagram shown in Fig. 8.

These out-of-plane and in-plane peaks agree with the peaks described in the following documents as XRD results of a DNTT thin film formed by a vapor deposition method, and therefore it is understood that in this Example, a thin film having the quality comparable to a DNTT thin film obtained by a vapor deposition method is formed.
(Document) T. Yamamoto, and K. Takimiya, J. Am. Chem. Soc., 2007, 129, 2224-2225
(Document) T. Someya et. al., Nature Comm., 3, 723

### <Evaluation of Organic Semiconductor Film - Semiconductor Characteristics>

In the region of 1.4 cmx1.4 cm of this organic semiconductor film, source/drain gold electrodes having a channel length of 200 µm and a channel width of 1.0 mm were formed by a vapor deposition method. In this way, 10 bottom-gate top-contact field-effect transistors were fabricated.

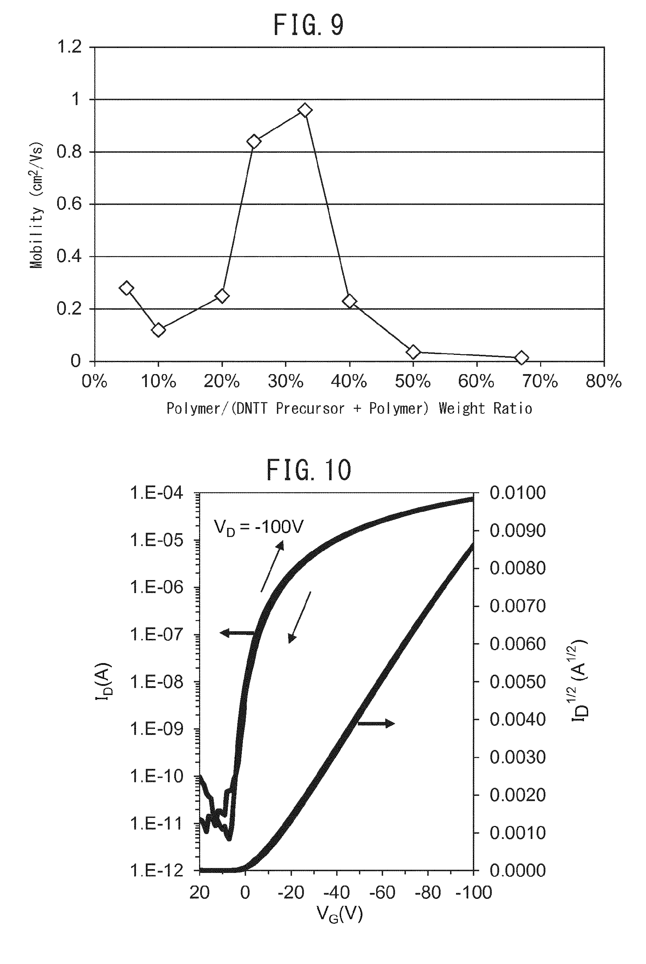

The characteristics of the transistor obtained in Example 2 are shown in Table 2 below and Fig. 9 together with the characteristics of transistors obtained in Examples 3 to 6 and Comparative Example 2.

Furthermore, Fig. 10 shows the transmission characteristics of this transistor. In Fig. 10, the relationship between a drain current (I_{D} (A) or I_{D}^{1/2} (A^{1/2)}) (ordinate) and a gate voltage (V_{G} (V)) (abscissa) when the drain voltage (V_{D}) is -100 V, is shown.

<<Examples 3 to 6 and Comparative Example 2>>

### <Fabrication of Transistor>

Bottom-gate top-contact field-effect transistors of Examples 3 to 9 were fabricated in the same manner as in Example 2 except that the ratio of the polymer in the organic semiconductor solution was changed to 5 mass% (Example 7), 10 mass% (Example 8), 20 mass% (Example 9), 25 mass% (Example 3), 40 mass% (Example 4), 50 mass% (Example 5), and 67 mass% (Example 6), from 33 mass%. In addition, a bottom-gate top-contact field-effect transistor of Comparative Example 2 was fabricated in the same manner as in Example 2 except that a polymer was not used in the organic semiconductor solution, i.e., the ratio of the polymer in the organic semiconductor solution was changed to 0 mass% from 33 mass%.

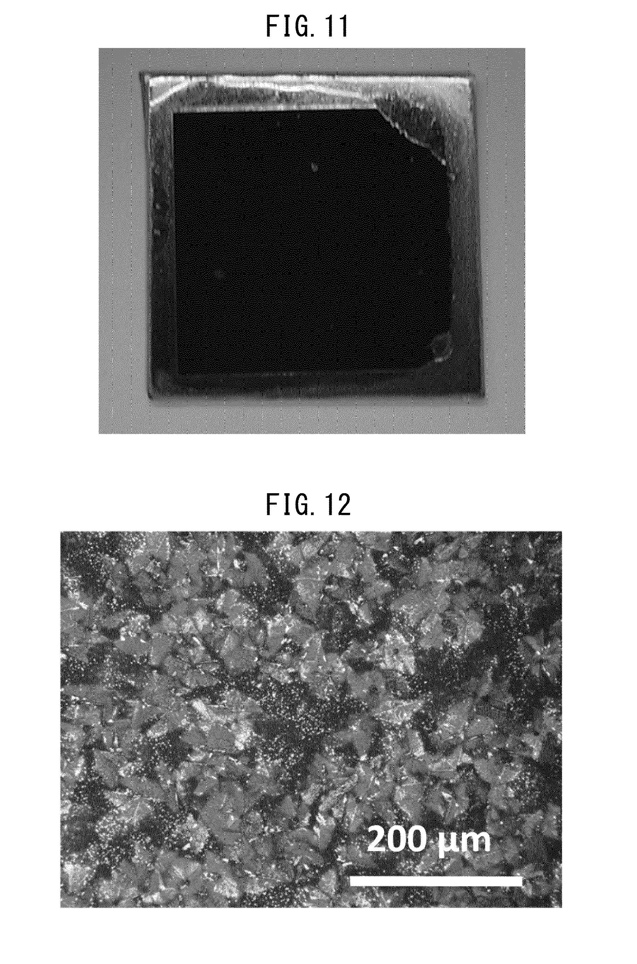

In Comparative Example 2 where the organic semiconductor solution does not contain a polymer, the organic semiconductor solution was, as shown in Fig. 11, accumulated at the edge part of the hydrophobic treated silicon wafer and a green film composed of a DNTT precursor could not be formed on the silicon wafer, failing in the fabrication of a transistor.

### <Evaluation of Organic Semiconductor Film - Semiconductor Characteristics>

The characteristics of the transistors obtained in Examples 3 to 9 and Comparative Example 2 are shown in Table 2 below and Fig. 9 together with the characteristics of the transistor obtained in Example 2. Incidentally, the value set forth in the parenthesis with regard to the ratio of the polymer is the mass fraction of the polymer based on the total of the polymer and the organic semiconductor when a DNTT precursor (molecular weight: 513.62) obtained by adding N-phenylmaleimide (molecular weight: 340.46) to DNTT (molecular weight: 173.16) is deprived of all N-phenylmaleimide and returned to DNTT.

[Table 2]

**Table 2**

| | Ratio of Polymer (mass%) | Mobility (cm²/Vs) | | | | On-Off Current Ratio |
|---|---|---|---|---|---|---|
| | | average | Maximum | Minimum | Standard Deviation | |
| Example 7 | 5% | 0.28 | 0.53 | 0.046 | 0.174 | 10⁵-10⁶ |
| Example 8 | 10% (6.6%) | 0.12 | 0.25 | 0.057 | 0.066 | 10⁵-10⁶ |
| Example 9 | 20% (13.2%) | 0.25 | 0.142 | 0.018 | 0.099 | 10⁵-10⁶ |
| Example 3 | 25% (16.6%) | 0.84 | 0.95 | 0.73 | 0.082 | 10⁶-10⁷ |
| Example 2 | 33% (21.9%) | 0.96 | 1.07 | 0.76 | 0.103 | 10⁶-10⁷ |
| Example 4 | 40% (26.5%) | 0.23 | 0.38 | 0.11 | 0.106 | 10⁶-10⁷ |
| Example 5 | 50% (33.1%) | 0.036 | 0.053 | 0.005 | 0.025 | 10⁶ |
| Example 6 | 67% (44.4%) | 0.014 | 0.022 | 0.008 | 0.006 | 10⁶ |
| Comparative Example 2 | 0% (0%) | (film cannot be formed) | | | | |

It is seen from Table 2 and Fig. 9 that when the ratio of the polymer to the total of the polymer and the organic semiconductor precursor is between 20 mass% and 40 mass%, i.e., when the ratio of the polymer to the total of the polymer and the organic semiconductor is between 13.2 mass% and 26.5 mass%, an organic semiconductor film particularly having excellent characteristics is obtained.

### «Example 7»

### <Formation of Organic Semiconductor Film>

An organic semiconductor film was formed in the same manner as in Example 2 except that a methyl methacrylate resin (PMMA, Polymer Science, Mw=100k) was used in place of polystyrene.

### <Evaluation of Organic Semiconductor Film - Crystal Grain Size>

Fig. 12 shows a photograph when the organic semiconductor film obtained is observed from the top surface by a polarizing microscope. It was confirmed from Fig. 12 that the organic semiconductor film has a large crystal grain with a grain size of more than 10 µm throughout the substrate surface.

### <Evaluation of Organic Semiconductor Film - Semiconductor Characteristics>

In the region of 1.4 cm×1.4 cm of this organic semiconductor film, source/drain gold electrodes having a channel width of 200 µm and a channel length of 1.0 mm were formed by a vapor deposition method. In this way, 10 bottom-gate top-contact field-effect transistors were fabricated.

These 10 transistors obtained exhibited a uniform high mobility of more than 0.5 cm²/Vs (maximum mobility: 0.70 cm²/Vs, average mobility: 0.66 cm²/Vs) and a good on-off current ratio of 10⁶ to 10⁷.

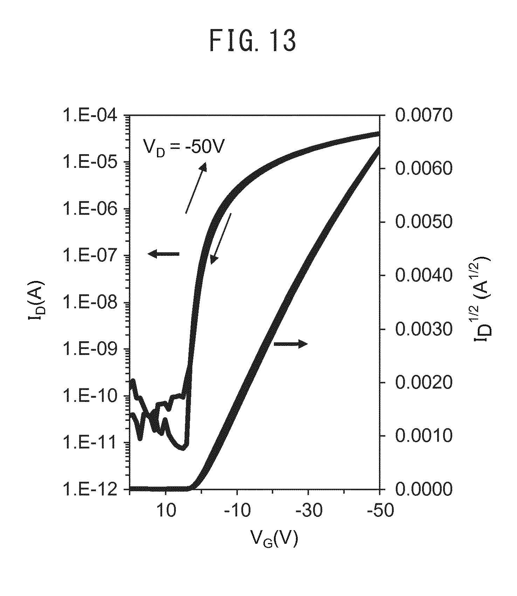

Furthermore, Fig. 13 shows the transmission characteristics of this transistor. In Fig. 13, the relationship between a drain current (I_{D} (A) or I_{D}^{1/2} (A^{1/2)}) (ordinate) and a gate voltage (V_{G} (V)) (abscissa) when the drain voltage (V_{D}) is -50 V, is shown.

### «Example 8»

### <Formation of Organic Semiconductor Film>

An organic semiconductor film was formed in the same manner as in Example 2 except that a polycarbonate resin (PC, TEIJIN Chemicals, Mw=25k) was used in place of polystyrene.

<Evaluation of Organic Semiconductor Film - Crystal Grain Size>

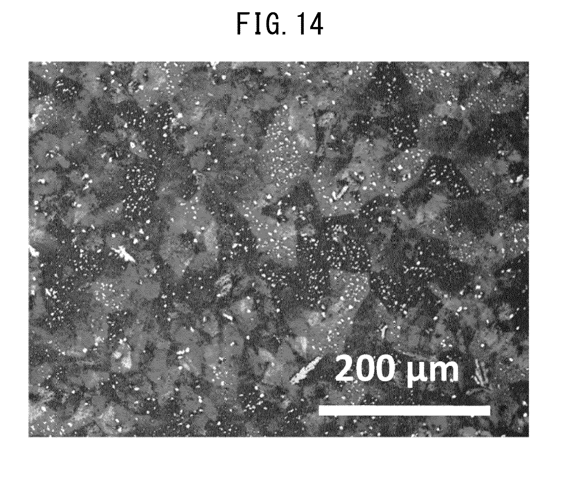

Fig. 14 shows a photograph when the organic semiconductor film obtained is observed from the top surface by a polarizing microscope. It was confirmed from Fig. 14 that the organic semiconductor film has a large crystal grain with a grain size of more than 10 µm throughout the substrate surface.

### <Evaluation of Organic Semiconductor Film - Semiconductor Characteristics>

In the region of 1.4 cm×1.4 cm of this organic semiconductor film, source/drain gold electrodes having a channel width of 200 µm and a channel length of 1.0 mm were formed by a vapor deposition method. In this way, 10 bottom-gate top-contact field-effect transistors were fabricated.

These 10 transistors obtained exhibited a uniform high mobility of more than 0.5 cm²/Vs (maximum mobility: 0.83 cm²/Vs, average mobility: 0.91 cm²/Vs) and a good on-off current ratio of 10⁶ to 10⁷.

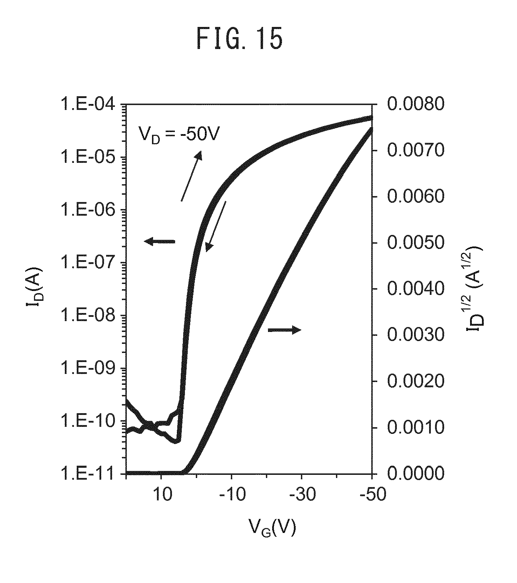

Furthermore, Fig. 15 shows the transmission characteristics of this transistor. In Fig. 15, the relationship between a drain current (I_{D} (A) or I_{D}^{1/2} (A^{1/2)}) (ordinate) and a gate voltage (V_{G} (V)) (abscissa) when the drain voltage (V_{D}) is -50 V, is shown.

### «Examples 9 to 13»

### <Formation of Organic Semiconductor Film>

Organic semiconductor films of Examples 9 to 13 were formed in the same manner as in Example 2 except that a polymer shown in Table 3 below was used in place of polystyrene.

<Evaluation of Organic Semiconductor Film - Crystal Grain Size>

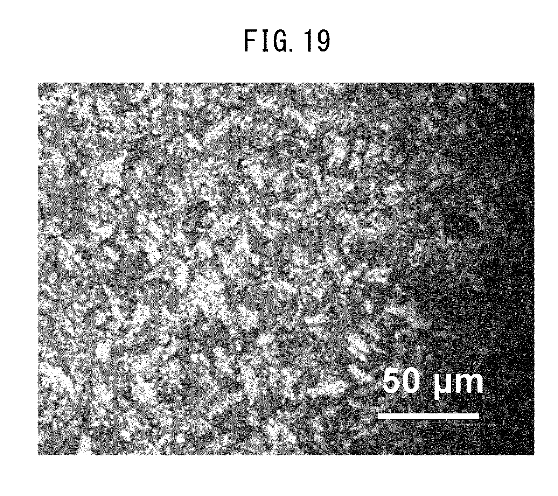

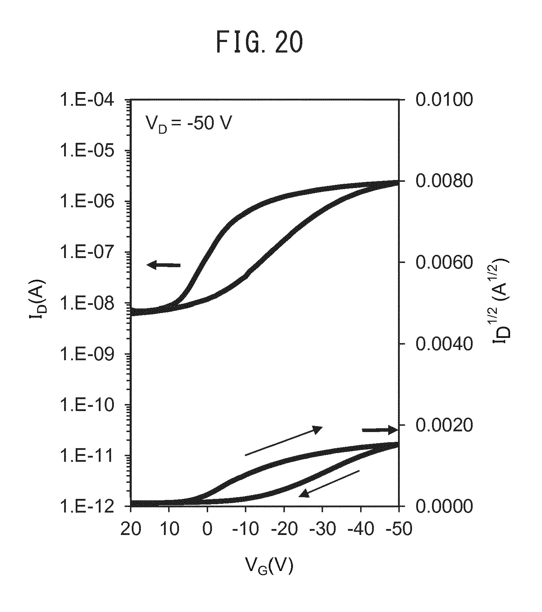

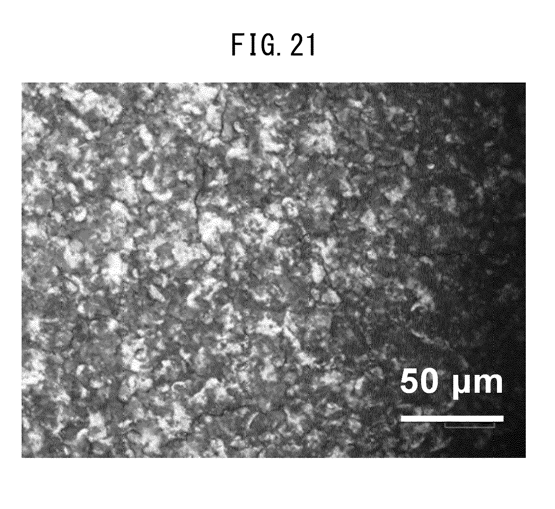

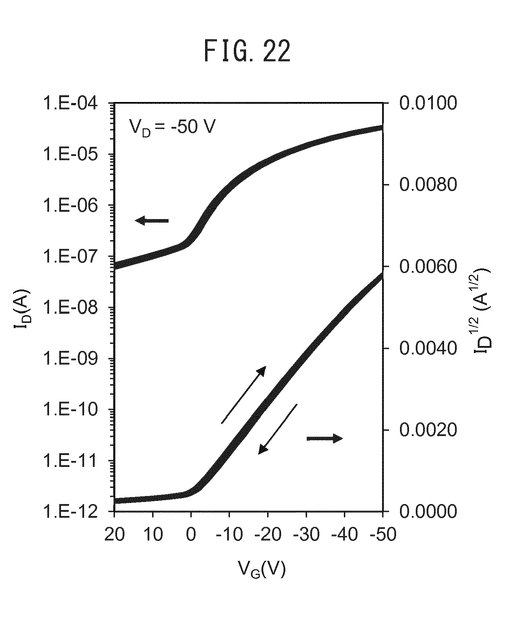

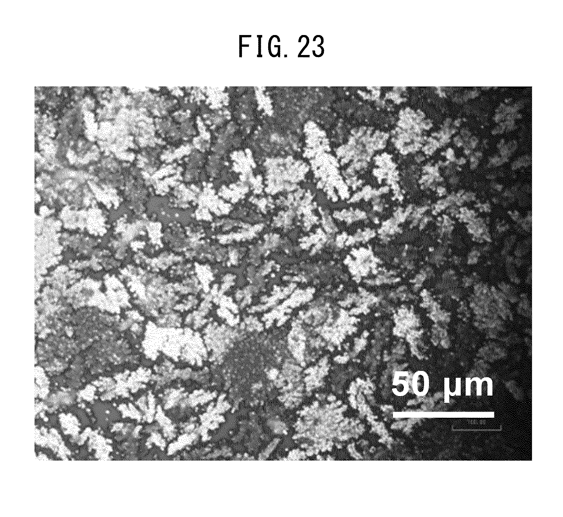

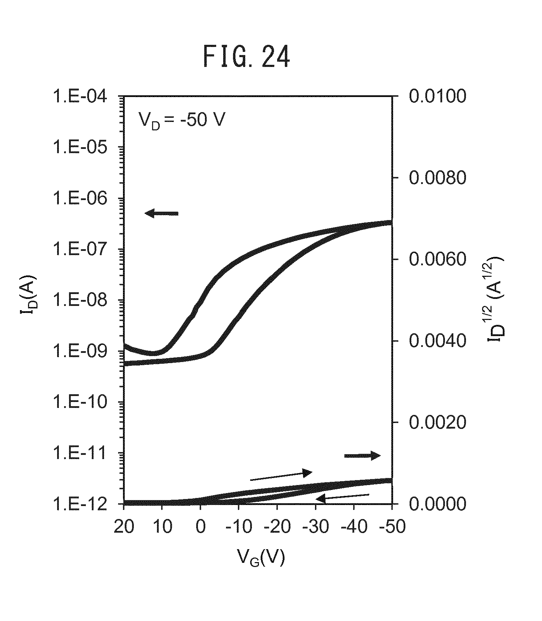

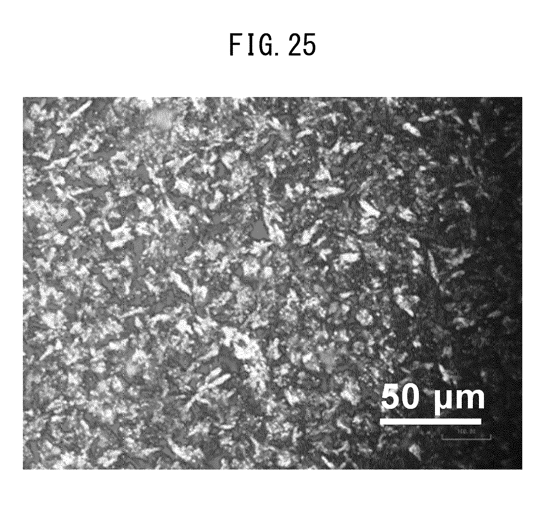

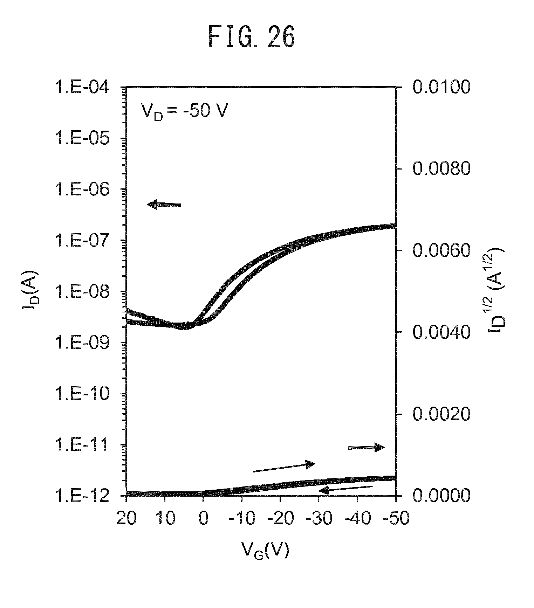

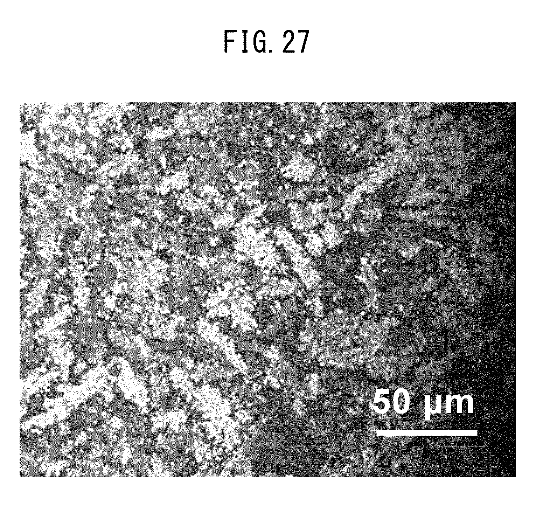

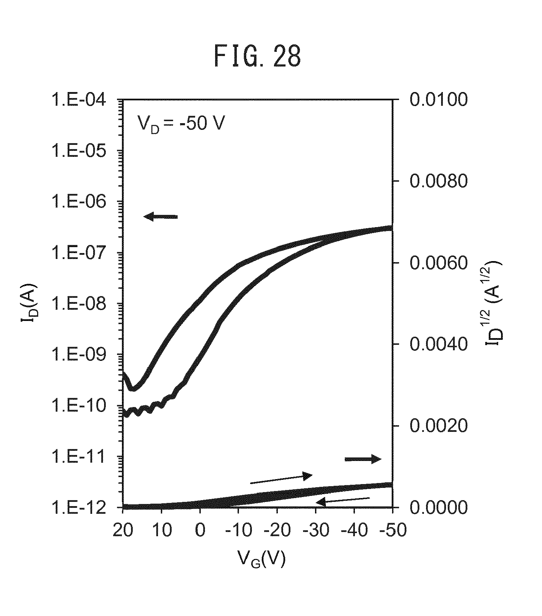

Figs. 19 to 28 show photographs when the organic semiconductor films obtained are observed from the top surface by a polarizing microscope, and the transmission characteristics of transistors obtained as in Example 8 by using these organic semiconductor films. Specifically, the relationship between the polymer used and the figure is as shown in Table 3 below.

[Table 3]

**Table 3**

| | Kind of Polymer | Polarizing Microscope Photograph | Transmission Characteristics of Transistor |
|---|---|---|---|
| Example 9 | triacetyl cellulose (TAC, Wako Pure Chemical) | Fig. 19 | Fig. 20 |
| Example 10 | polyvinyl acetate (PVAC, Aldrich, Mw=140k) | Fig. 21 | Fig. 22 |
| Example 11 | polyvinylpyrrolidone (PVP, Aldrich, Mw=29k) | Fig. 23 | Fig. 24 |
| Example 12 | polysulfone (PSF, Aldrich, Mw=16k) | Fig. 25 | Fig. 26 |
| Example 13 | poly(2,6-dimethyl-1,4-phenylene oxide) (PPO, Aldrich) | Fig. 27 | Fig. 28 |

From the polarizing microscope photographs of organic semiconductor films of Examples 9 to 13, the organic semiconductor film was confirmed to have a crystal grain throughout the substrate surface. In addition, from the transmission characteristics of transistors obtained using the organic semiconductor films of Examples 9 to 13, these organic semiconductor films were confirmed to function as a semiconductor film.

### «Reference Examples»

The following Reference Examples are intended to explain the synthesis method of an organic semiconductor compound of formula (I) where E₁ to E₄ are substituted. With regard to such an organic semiconductor compound, Patent Document 7 may be referred to.

In the following Reference Examples, the structure of the target compound was determined as needed by 1H-nuclear magnetic resonance spectrum (1H-NMR spectrum), mass spectrometry (MS), single-crystal structural analysis and gel permeation chromatography (GPC).

The devices used are as follows.
1H-NMR: ECA-500 of JEOL (500 MHz)
MS: Autoflex III (MALDI) of Bruker
Single-crystal structural analysis: RAXIS RAPIDS of Rigaku
GPC: LC-9101 of Japan Analytical Industry Co., Ltd. (column: JAIGEL-2H, JAIGEL-1H)

### «Reference Example 1»

Dinaphthothienothiophene (DNTT) (structural formula shown below, MW=340.46) was synthesized by the method described in Patent Document 1.

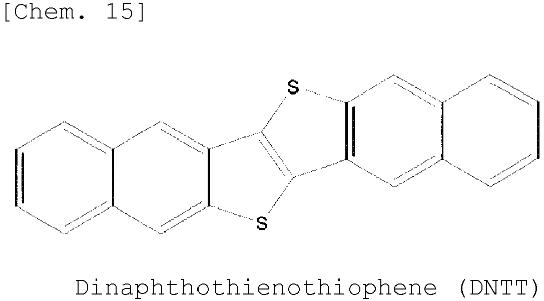

To 100 mL of mesitylene (i.e., 1,3,5-trimethylbenzene) containing 1,000 mg (2.93 mmol) of DNTT above, 2,341 mg (14.65 mmol) of bromine (Br₂, MW=159.8) was added, and the reaction temperature was kept at 40°C for 4 hours. Thereafter, the reaction solution was allowed to cool to obtain di-bromine-substituted dinaphthothienothiophene (di-Br-substituted DNTT) (structural formula shown below, Mw=498.25, 1431 mg, 2.87 mmol, yield: 98.1%). Incidentally, the reaction product was purified in chloroform.

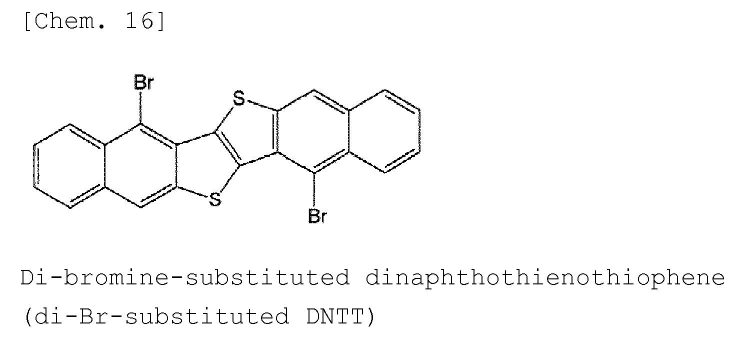

Incidentally, the substitution position of bromine in the di-Br-substituted DNTT was judged from the position of the triisopropylsilyl (TIPS) group by single-crystal structural analysis of di-TIPS-substituted DNTT in Reference Example 2.

The ¹H-NMR and MS results of the obtained di-Br-substituted DNTT are shown below.

¹H-NMR (500 MHz, CDCl3, 50°C): δ8.47 (d, J=8.3 Hz, 2H), 8.44 (s, 2H), 7.97 (d, J=8.3 Hz, 2H), 7.66 (t, J=8.3 Hz, 2H), 7.59 (t, J=8.3 Hz, 2H).

MS (m/z): 497.513 (positive ion observation) (Exact Mass: 495.86).

### <<Reference Example 1A>>

To a flask, 100.7 mg (0.296 mmol) of DNTT synthesized as in Reference Example 1A and 17.7 mg (0.133 mmol) of aluminum chloride were added, and nitrogen purging was performed three times. Thereafter, 5.0 ml of chloroform was added and after cooling to 0°C, 78.7 mg (0.589 mmol) of N-chlorosuccinimide was added, followed by stirring for 1.5 hours.

After confirming that N-chlorosuccinimide as a raw material disappeared by mass spectrometry (MS), 5.0 ml of water was added to terminate the reaction. The reaction product was filtered to obtain di-chlorine-substituted dinaphthothienothiophene (di-Cl-substituted DNTT) (structural formula shown below, Mw=409.35, 116.0 mg, 0.28 mmol, yield: 95.8%). Incidentally, the reaction product was purified in chloroform.

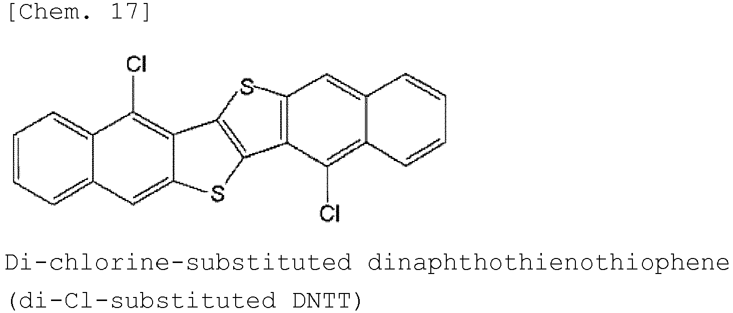

The MS result of the obtained di-Cl-substituted DNTT is shown below.

MS (m/z): 407.822 (positive ion observation) (Exact Mass: 407.96).

### <<Reference Example 2>>

The di-Br-substituted DNTT synthesized in Reference Example 1 was subjected to introduction of a triisopropylsilyl (TIPS) group by the Sonogashira coupling method.

Specifically, 191.3 mg of Pd(PPh₃)₂Cl₂ (Mw=701.90), 134.1 mg of CuI (Mw=190.45), 0.706 mL of diisopropylamine (Mw=101.20), 791.6 mg of CsCO₃ (Mw=325.82) and 1.698 mL of triisopropylsilylacetylene (Mw=182.38) were added to 500 mg (1.0 mmol) of di-Br-substituted DNTT (Mw=498.25), and deaeration under reduced pressure and nitrogen purging were performed three times. Thereafter, 35 mL of N,N-dimethylformamide (N,N-DMF) was introduced and after again performing deaeration under pressure and nitrogen purging three times, the system was stirred at 120°C over 20 hours, thereby allowing the reaction to proceed.

By this reaction, 479.1 mg (68.3 mmol, yield: 68.0%) of di-triisopropylsilylacetylene-substituted dinaphthothienothiophene (di-TIPS-substituted DNTT, structural formula shown below) (Mw=701.19) was obtained. The solubility of the obtained di-TIPS-substituted DNTT in chloroform was 0.2 wt%. Incidentally, DNTT used as a raw material was substantially not dissolved in chloroform.

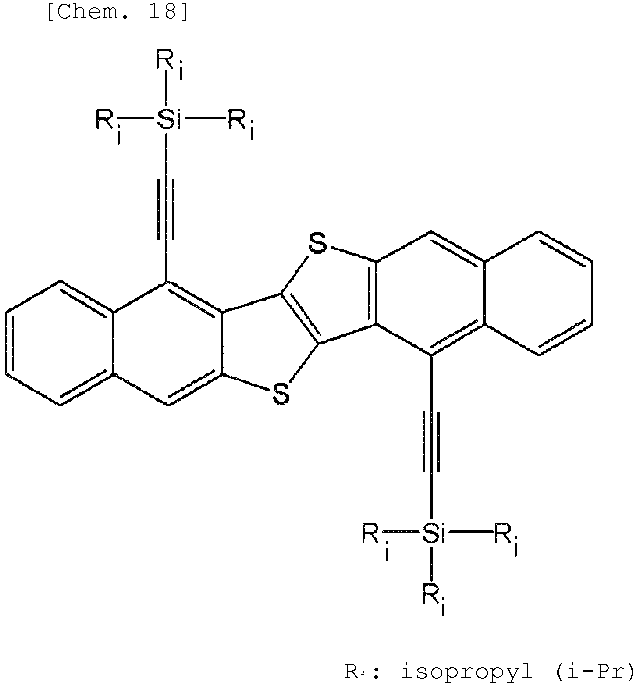

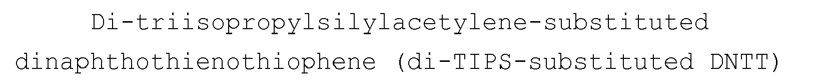

The ¹H-NMR and MS results of the obtained di-TIPS-substituted DNTT are shown below.

¹H-NMR (500 MHz, CDCl₃) : δ8.61 (d, J=8.0 Hz, 2H), 8.41 (s, 2H), 7.97 (d, J=8.0 Hz, 2H), 7.63 (dd, J=8.0 Hz, 8.0 Hz 2H), 7.57 (dd, J=8.0 Hz, 8.0 Hz, 2H), 1.40-1.47 (m, 6H), 1.34 (d, J=6.9 Hz, 36H)

MS (m/z): 700.3 (Exact Mass: 700.30)

The single crystal structural analysis results of the obtained di-TIPS-substituted DNTT are shown below.

a=8.2044 (5) Å
b=8.4591 (6) Å
c=14.488 (1) Å
α=88.475 (4)°
β=89.336 (3)°
γ=89.555 (4)°
v=1005.1 (1) Å³

Also, the molecular structure ORTEP (Oak Ridge Thermal Ellipsoid Plot) drawing based on single-crystal structural analysis and the crystal packing (stereo) diagram of this di-TIPS-substituted DNTT are depicted in Figs. 3 and 4, respectively.

### <<Reference Example 2A>>

The di-triisopropylsilylacetylene-substituted DNTT (di-TIPS-substituted DNTT) synthesized in Reference Example 2 was hydrogen-reduced to synthesize di-triisopropylsilylethane-substituted DNTT.

Specifically, to a 200-ml flask, 304.6 mg (0.43 mmol) of di-triisopropylsilylacetylene-substituted DNTT (Mw=701.18), 60 ml of toluene and 79.2 mg of 10% Pd/C were added, and purging with hydrogen was performed three times. The system was stirred at 60°C for 15 hours in a hydrogen atmosphere, thereby allowing the reaction to proceed.

By this reaction, di-triisopropylsilylethane-substituted DNTT (structural formula shown below, Mw=709.37, 296.1 mg, 0.42 mmol, yield: 96.1%) was obtained. The solubility of the obtained di-triisopropylsilylethane-substituted DNTT in chloroform was 0.51 wt%. Incidentally, DNTT used as a raw material was substantially not dissolved in chloroform.

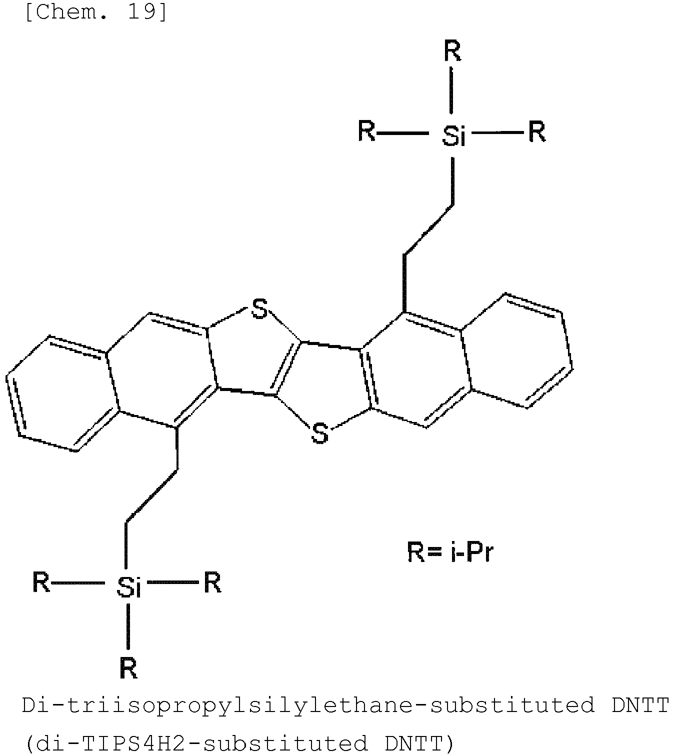

The ¹H-NMR and MS results of the obtained di-triisopropylsilylethane-substituted DNTT are shown below.

¹H-NMR (500 MHz, CDCl3, 50°C) : δ8.33 (s, 2H), 7.26 (d, J=8.6 Hz, 2H), 7.97 (d, J=8.6 Hz, 2H), 7.57 (dd, J=8.6 Hz, 8.6 Hz, 2H), 7.53 (dd, J=8.3 Hz, 8.3 Hz, 2H), 3.76-3.72 (m, 8H), 1.39-1.32 (m, 6H), 1.30-1.19 (m, 36H).

MS (m/z): 708.322 (positive ion observation) (Exact Mass: 708.367).

### «Reference Example 3»

Di-TIPS-substituted DNTT (Mw=701.19) obtained in Reference Example 2 was dissolved in chloroform at a concentration of 0.2 wt% to prepare a solution for the manufacture of a semiconductor device.

Next, an n-doped silicone wafer with an SiO₂ oxide film of 300 nm (surface resistance: 1⁻¹⁰ Ω·cm) was subjected to a UV-ozone treatment for 20 minutes (Eye UV-Ozone Cleaning System OC-250615-D+A, Eye Graphics Co., Ltd.). Also, a toluene solution containing 10 mmol of 1,1,1,3,3,3-hexamethyldisilazane (HMDS) was prepared, and the silicon substrate subjected the UV ozone treatment was dipped in this solution over 24 hours, thereby performing a hydrophobing treatment of the silicon substrate. Thereafter, source/drain gold electrodes having a channel width of 50 µm and a channel length of 1.5 mm were produced by the vacuum deposition method (resistance-heating vapor deposition apparatus: SVC-700 TM/700-2, Sanyu Electron Co., Ltd.).

While heating the silicon substrate at 40°C, the solution for the manufacture of a semiconductor device was dropped on the channel portion to volatilize the solvent, and thereby form a thin layer composed of di-TIPS-substituted DNTT. The thus-produced device was heat-treated at 70°C over 1 hour in a vacuum to dry and remove the chloroform solvent and thereby manufacture an organic semiconductor device.

The obtained organic semiconductor device was measured for the organic semiconductor characteristics and found to exhibit p-type semiconductor behavior. Also, in this organic semiconductor device, the carrier mobility was 1×10⁻³ cm²/Vs, the on/off ratio was 10⁵, and the threshold voltage was -26 V. Fig. 5 illustrates the transmission property of FET characteristics. Fig. 5 shows the relationship between the drain current (I_{D}(A) or I_{D}^{1/2} (A^{1/2})) (ordinate) and the gate voltage (V_{G}(V)) (abscissa) when the drain voltage (V_{D}) is -80 V.

### «Reference Example 4»

Dinaphthothienothiophene (DNTT) (MW=340.46) was synthesized in the same manner as in Reference Example 1.

To 500 mL of mesitylene (i.e., 1,3,5-trimethylbenzene) containing 5,000 mg (14.65 mmol) of DNTT above, 12.68 g (73.25 mmol) of N-phenylmaleimide (MW=173.17) was added, and the reaction temperature was kept at 160°C for 4 hours. Thereafter, the system was allowed to cool and through separation and purification, 376 mg (0.73 mmol, yield: 4.9%) of dinaphthothienothiophene-N-phenylmaleimide monoadduct in which one N-phenylmaleimide was added to DNTT (DNTT-PMI monoadduct, a mixture of Endo form and Exo form which are stereoisomers) (structural formula shown below, Mw=513.63) was obtained. Incidentally, stereoisomers were separated by HPLC to obtain 132 mg of Endo form and 151 mg of Exo form.

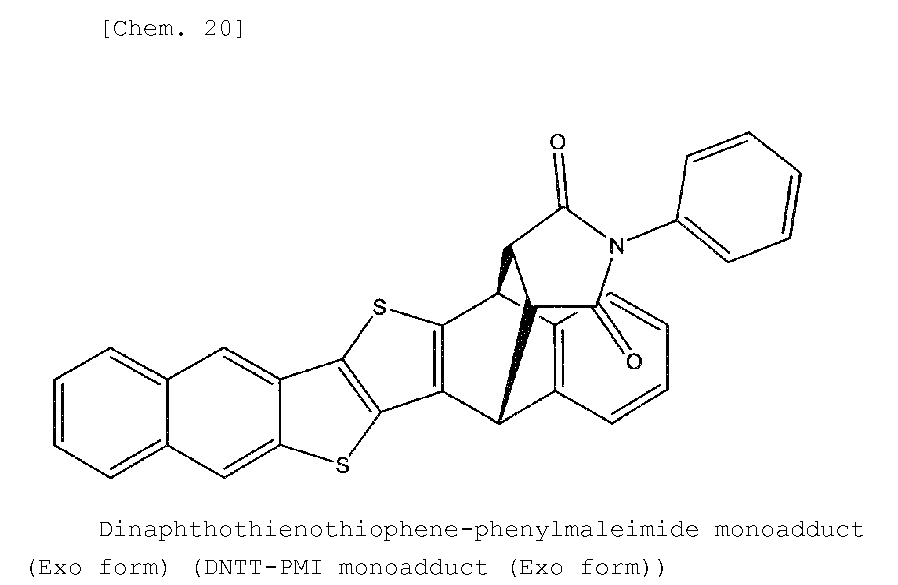

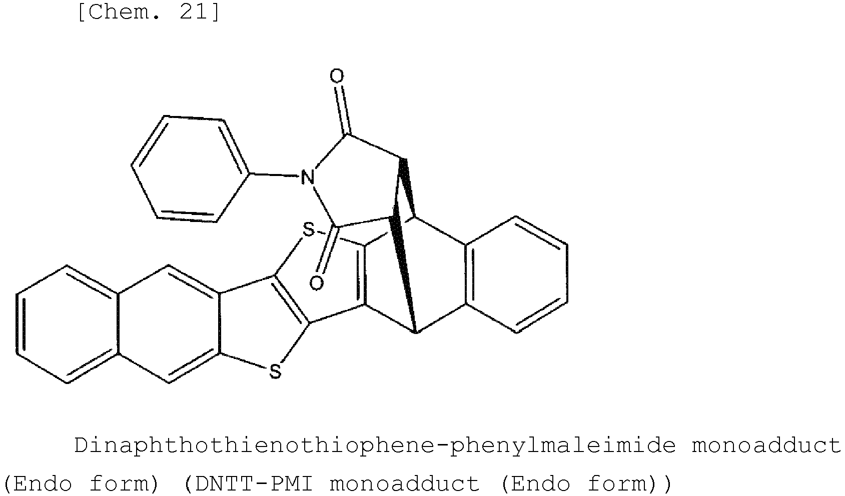

To 50 mL of mesitylene containing 151 mg (0.29 mmol) of the DNTT-PMI monoadduct (Exo form) above, 235 mg (1.47 mmol) of bromine (Br₂, MW=159.8) was added, and the reaction temperature was kept at 40°C for 1 hour. Thereafter, the system was allowed to cool, as a result, 186 mg (0.276 mmol, yield: 95.2%) of di-bromine-substituted dinaphthothienothiophene-N-phenylmaleimide monoadduct (di-Br-substituted DNTT-PMI monoadduct) (structural formula shown below, Mw=673.44) was obtained.

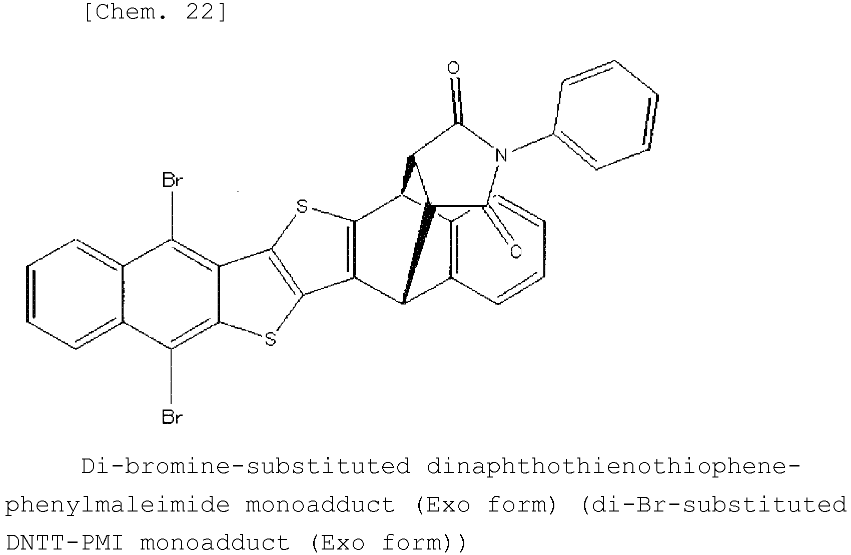

The ¹H-NMR and MS results of the obtained di-Br-substituted DNTT-PMI monoadduct are shown below.

¹H-NMR (500 MHz, CDCl₃) : δ8.39 (d, J=7.7 Hz, 1H), 8.29 (d, J=7.7 Hz, 1H), 7.67 (dd, J=7.7 Hz, 1H), 7.64 (dd, J=7.7 Hz, 1H), 7.41-7.44 (m, 2H), 7.31-7.32 (m, 3H), 7.27-7.29 (m, 2H), 6.52-6.54 (m, 2H), 5.25 (d, J=3.2 Hz, 1H), 5.23 (d, J=3.2 Hz, 1H), 3.59 (dd, J=3.2 Hz, 8.3 Hz, 1H), 3.55 (dd, J=3.2 Hz, 8.3 Hz, 1H).

MS (m/z): 497.513 (Exact Mass: 670.92)

Incidentally, it is presumed that in MS, di-Br-substituted DNTT (Exact Mass: 497.86) formed as a result of desorption of N-phenylmaleimide from the di-Br-substituted DNTT-PMI monoadduct was observed.

«Reference Example 5»

The di-Br-substituted DNTT-PMI monoadduct synthesized in Reference Example 4 was subjected to introduction of a triisopropylsilyl (TIPS) group by the Sonogashira coupling method.

Specifically, 28.1 mg of Pd(PPh₃)₂Cl₂ (Mw=701.90), 20.0 mg of CuI (Mw=190.45), 0.11 mL of diisopropylamine (Mw=101.20) and 0.1 mL of triisopropylsilylacetylene (Mw=182.38) were added to 100 mg (0.148 mmol) of di-Br-substituted DNTT-PMI monoadduct (Mw=673.44), and deaeration under reduced pressure and nitrogen purging were performed three times. Thereafter, 7 mL of N,N-dimethylformamide (N,N-DMF)was introduced and after again performing deaeration under pressure and nitrogen purging three times, the system was stirred at 120°C over 20 hours, thereby allowing the reaction to proceed.

By this reaction, 74.9 mg (85.4 mmol, yield: 57.7%) of di-triisopropylsilylacetylene-substituted dinaphthothienothiophene-phenylmaleimide monoadduct (exo form) (di-TIPS-substituted DNTT-PMI monoadduct (exo form)) (structural formula shown below, Mw=876.37) was obtained.

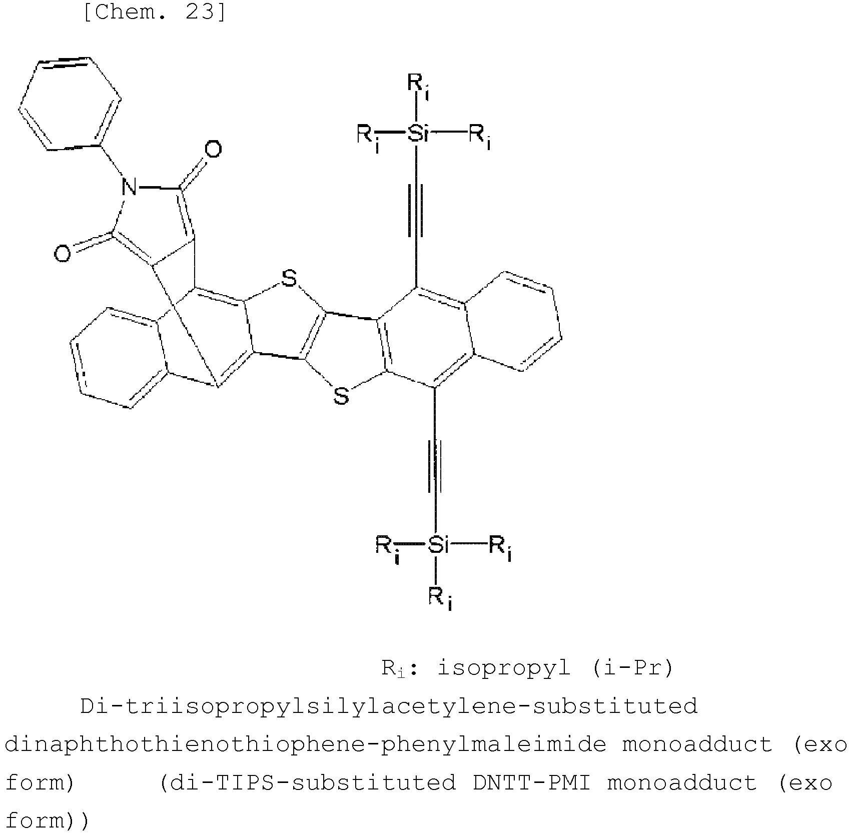

The ¹H-NMR and MS results of the obtained di-TIPS-substituted DNTT-PMI monoadduct (exo form) are shown below.

¹H-NMR (500 MHz, CDCl₃) : δ8.52-8.54 (m, 1H), 8.43-8.45 (m, 1H), 7.60-7.64 (m, 2H), 7.43-7.46 (m, 2H), 7.31-7.33 (m, 3H), 7.25-7.29 (m, 2H), 6.52-6.54 (m, 2H), 5.29 (d, J=3.4 Hz, 1H), 5.21 (d, J=3.4 Hz, 1H), 3.62 (dd, J=3.4 Hz, 8.3 Hz, 1H), 3.56 (dd, J=3.4 Hz, 8.3 Hz, 1H), 1.36-1.43 (m, 6H), 1.31 (d, J=2.9 Hz, 12H), 1.30 (d, J=4.0 Hz, 24H)

MS (m/z): 873.078 (Exact Mass: 875.37).

### <<Reference Example 6>>

The di-Br-substituted DNTT synthesized in Reference Example 1 was reacted with 1-decyne by the Sonogashira coupling method to synthesize di-1-decyne-substituted DNTT.

Specifically, 761.1 mg (1.08 mmol) of Pd(PPh₃)₂Cl₂ (Mw=701.90), 577 mg (3.03 mmol) of CuI (Mw=190.45) and 3.20 g (9.82 mmol) of Cs₂CO₃ (Mw=325.8) were added to 1000 mg (2.01 mmol) of di-Br-substituted DNTT (Mw=498.25), and deaeration under reduced pressure and nitrogen purging were performed five times.

Thereafter, 70 ml of dimethylformamide, 1.41 ml (10.0 mmol) of diisopropylamine (Mw=101.2, d=0.72 g/cm³) and 2.78 ml (15.4 mmol) of 1-decyne (Mw=138.25, d=0.77 g/cm³) were added and after again performing deaeration under reduced pressure and nitrogen purging five times, the system was stirred at 120°C over 15.5 hours, thereby allowing the reaction to proceed.

By this reaction, di-1-dysine-substituted DNTT (structural formula shown below, Mw=612.93, 20.5 mg, 0.033 mmol, yield: 1.6%) was obtained. The solubility of the obtained di-1-dysine-substituted DNTT in chloroform was 2.3 wt%. Incidentally, DNTT used as a raw material was substantially not dissolved in chloroform.

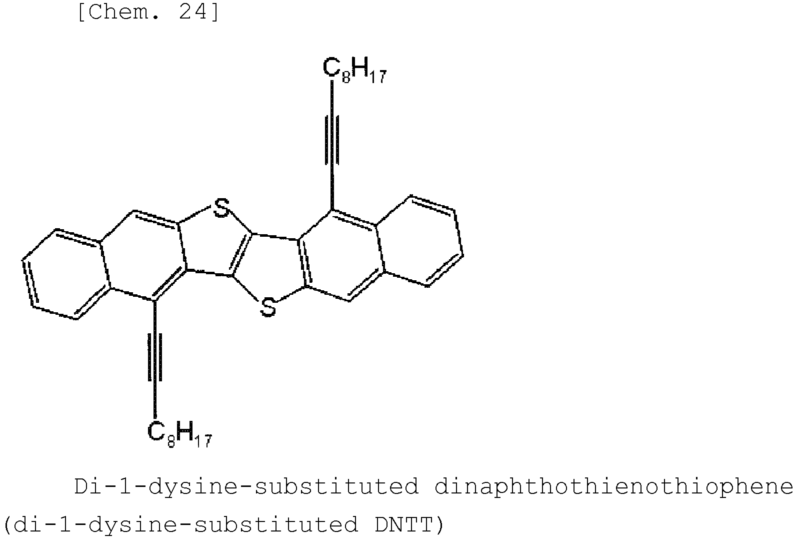

The ¹H-NMR and MS results of the obtained di-1-dysine-substituted DNTT are shown below.

¹H-NMR (500 MHz, CDCl3, 50°C): δ8.45 (d, J=8.3 Hz, 2H), 8.25 (s, 2H), 7.86 (d, J=8.3 Hz, 2H), 7.54 (dd, J=8.3 Hz, 6.9 Hz, 2H), 7.48 (dd, J=8.3 Hz, 6.9 Hz, 2H), 2.81 (t, J=7.2 Hz, 4H), 1.90 (tt, J=7.2 Hz, 7.2 Hz, 4H), 1.65 (tt, J=7.2 Hz, 7.2 Hz, 4H), 1.31-1.48 (m, 16H), 0.89 (t, J=7.2 Hz, 6H).

MS (m/z): 612.284 (positive ion observation) (Exact Mass: 612.288).

### «Reference Example 7»

The di-Br-substituted DNTT synthesized in Reference Example 1 was reacted with 1-tetradecyne by the Sonogashira coupling method to synthesize di-1-tetradecyne-substituted DNTT.

Specifically, 830.0 mg (1.17 mmol) of Pd(PPh₃)₂Cl₂ (Mw=701.90), 590.0 mg (3.12 mmol) of CuI (Mw=190.45) and 1.62g (4.98 mmol) of Cs₂CO₃ (Mw=325.8) were added to 1000 mg (2.01 mmol) of di-Br-substituted DNTT (Mw=498.25), and deaeration under reduced pressure and nitrogen purging were performed five times.

Thereafter, 70 ml of dimethylformamide, 1.41 ml (10.0 mmol) of diisopropylamine (Mw=101.2, d=0.72 g/cm³) and 4.19 ml (17.0 mmol) of 1-tetradecyne (Mw=194.36, d=0.79 g/cm³) were added and after again performing deaeration under reduced pressure and nitrogen purging five times, the system was stirred at 120°C over 12 hours, thereby allowing the reaction to proceed.

By this reaction, di-1-tetradysine-substituted DNTT (structural formula shown below, Mw=612.93, 156.04 mg, 0.207 mmol, yield: 10.3%) was obtained. The solubility of the obtained di-1-tetradysine-substituted DNTT in chloroform was 6.5 wt%. Incidentally, DNTT used as a raw material was substantially not dissolved in chloroform.

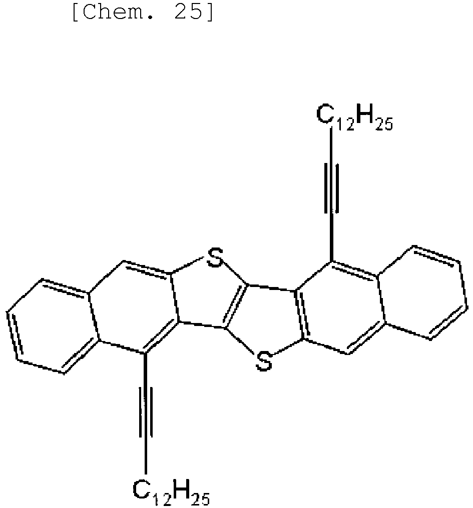

The ¹H-NMR and MS results of the obtained di-1-tetradysine-substituted DNTT are shown below.

¹H-NMR (500 MHz, CDCl3, 50°C) : δ8.38 (d, J=8.3 Hz, 2H), 8.14 (s, 2H), 7.78 (d, J=8.3 Hz, 2H), 7.48 (dd, J=8.3 Hz, 6.6 Hz, 2H), 7.42 (dd, J=8.3 Hz, 6.6 Hz, 2H), 2.76 (t, J=7.2 Hz, 4H), 1.86 (tt, J=7.2 Hz, 7.2 Hz, 4H), 1.62 (tt, J=7.2 Hz, 7.2 Hz, 4H), 1.24-1.46 (m, 32H), 0.86 (t, J=7.2 Hz, 6H).

MS (m/z): 724.411 (positive ion observation) (Exact Mass: 724.414).

### <<Reference Example 7A>>

The di-Br-substituted DNTT synthesized in Reference Example 1 was reacted with 1-tetradecyne by using Negishi coupling in place of the Sonogashira coupling method used in Reference Example 7 to synthesize di-1-tetradecyne-substituted DNTT.

Specifically, to a 10-ml flask, 0.21 ml (0.85 mmol) of 1-tetradesyne (Mw=194.36, d=0.79 g/cm³) and 3.5 ml of toluene were added, and deaeration under reduced pressure and nitrogen purging were performed three times. After cooling to 0°C, 0.53 ml (0.859 mmol) of a hexane solution of n-BuLi was added and then, the temperature was raised to room temperature.

Thereafter, 215.3 mg (0.853 mmol) of ZnCl₂ (TMEDA) (Mw=252.50), 50 mg (0.10 mmol) of di-Br-substituted DNTT (Mw=498.25) and 35.2 mg (0.05 mmol) of Pd(PPh₃)₂Cl₂ (Mw=701.90) were added, and deaeration under reduced pressure and nitrogen purging were performed three times. The system was stirred at 100°C over 12 hours, thereby allowing the reaction to proceed.

By this reaction, di-1-tetradesyne-substituted DNTT (structural formula shown above, Mw=612.93, 8.9 mg, 0.012 mmol, yield: 12.2%) was obtained.

### «Reference Example 8»

The di-Br-substituted DNTT synthesized in Reference Example 1 was reacted with 1-octadecyne by the Sonogashira coupling method to synthesize di-1-octadecyne-substituted DNTT.

Specifically, 820.0 mg (1.17 mmol) of Pd(PPh₃)₂Cl₂ (Mw=701.90), 590.0 mg (3.12 mmol) of CuI (Mw=190.45) and 1.58g (4.85 mmol) of Cs₂CO₃ (Mw=325.8) were added to 1010 mg (2.02 mmol) of di-Br-substituted DNTT (Mw=498.25), and deaeration under reduced pressure and nitrogen purging were performed five times.

Thereafter, 70 ml of dimethylformamide, 1.41 ml (10.0 mmol) of diisopropylamine (Mw=101.2, d=0.72 g/cm³) and 5.34 ml (16.8 mmol) of 1-octadecyne (Mw=250.46, d=0.79 g/cm³) were added and after again performing deaeration under reduced pressure and nitrogen purging five times, the system was stirred at 120°C over 14 hours, thereby allowing the reaction to proceed.

By this reaction, di-1-octadecyne-substituted DNTT (structural formula shown below, Mw=836.54, 54.2 mg, 0.064 mmol, yield: 3.2%) was obtained.

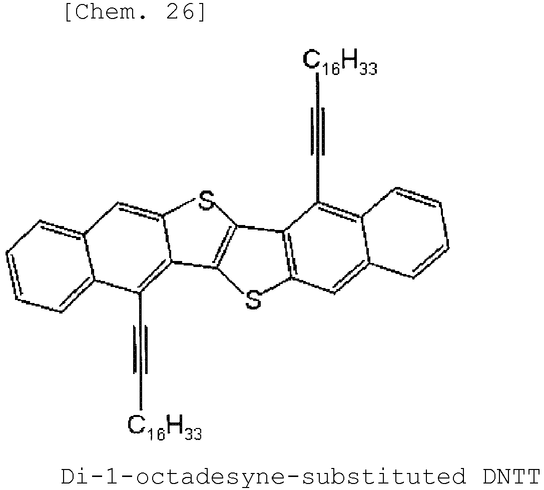

### <<Reference Example 8A>>

The di-Br-substituted DNTT synthesized in Reference Example 1 was reacted with 1-tetradecyne by using Negishi coupling in place of the Sonogashira coupling method used in Reference Example 8 to synthesize di-1-octadecyne-substituted DNTT.

Specifically, to a 10-ml flask, 0.26 ml (0.85 mmol) of 1-octadesyne (Mw=250.46, d=0.80 g/cm³) and 3.5 ml of toluene were added, and deaeration under reduced pressure and nitrogen purging were performed three times. After cooling to 0°C, 0.53 ml (0.859 mmol) of a hexane solution of n-BuLi was added and then, the temperature was raised to room temperature.

Thereafter, 216.1 mg (0.856 mmol) of ZnCl₂ (TMEDA) (Mw=252.50), 49.7 mg (0.10 mmol) of di-Br-substituted DNTT (Mw=498.25) and 35.6 mg (0.05 mmol) of Pd(PPh₃)₂Cl₂ (Mw=701.90) were added, and deaeration under reduced pressure and nitrogen purging were performed three times. The system was stirred at 100°C over 12 hours, thereby allowing the reaction to proceed.

By this reaction, di-1-octadesyne-substituted DNTT (structural formula shown above, Mw=836.54, 3.8 mg, 0.004 mmol, yield: 4.5%) was obtained. The solubility of the obtained di-1-tetradesyne-substituted DNTT in chloroform was 1.67 wt%. Incidentally, DNTT used as a raw material was substantially not dissolved in chloroform.

The ¹H-NMR and MS results of the obtained di-1-octadesyne-substituted DNTT are shown below.

¹H-NMR (500 MHz, CDCl3, 50°C): δ8.48 (d, J=8.3 Hz, 2H), 8.30 (s, 2H), 7.89 (d, J=8.3 Hz, 2H), 7.56 (dd, J=8.3 Hz, 6.6 Hz, 2H), 7.51 (dd, J=8.3 Hz, 6.6 Hz, 2H), 2.83 (t, J=7.2 Hz, 4H), 1.91 (tt, J=7.2 Hz, 7.2 Hz, 2H), 1.66 (tt, J=7.2 Hz, 7.2 Hz, 2H), 1.24-1.46 (m, 48H), 0.87 (t, J=7.2 Hz, 6H).

MS (m/z): 836.539 (positive ion observation) (Exact Mass: 836.537).

### «Reference Example 9»

The di-Br-substituted DNTT synthesized in Reference Example 1 was reacted with trimethylsilylacetylene (TMS) by the Negishi coupling method to synthesize di-TMS-substituted DNTT.

To a 200-ml flask, 1.68 g (17.10 mmol) of trimethylsilylacetylene (Mw=98.22, d=0.70 g/cm³) and 70 ml of toluene were added, and deaeration under reduced pressure and nitrogen purging were performed three times. After cooling to 0°C, 10.3 ml (16.68 mmol) of a hexane solution of n-BuLi was added and then, the temperature was raised to room temperature.

Thereafter, 4.31 g (17.06 mmol) of ZnCl₂ (TMEDA) (Mw=252.50), 1.0 g (2.00 mmol) of di-Br-substituted DNTT (Mw=498.25) and 702.5 mg (1.00 mmol) of Pd(PPh₃)₂Cl₂ (Mw=701.90) were added, and deaeration under reduced pressure and nitrogen purging were performed three times. The system was stirred at 100°C over 12 hours, thereby allowing the reaction to proceed.

By this reaction, di-trimethylsilylacetylene-substituted DNTT (di-TMS-substituted DNTT) (structural formula shown above, Mw=532.87, 536.30 mg, 1.00 mmol, yield: 50.1%) was obtained. The solubility of the obtained di-trimethylsilylacetylene-substituted DNTT in chloroform was 0.05 wt%. Incidentally, DNTT used as a raw material was substantially not dissolved in chloroform.

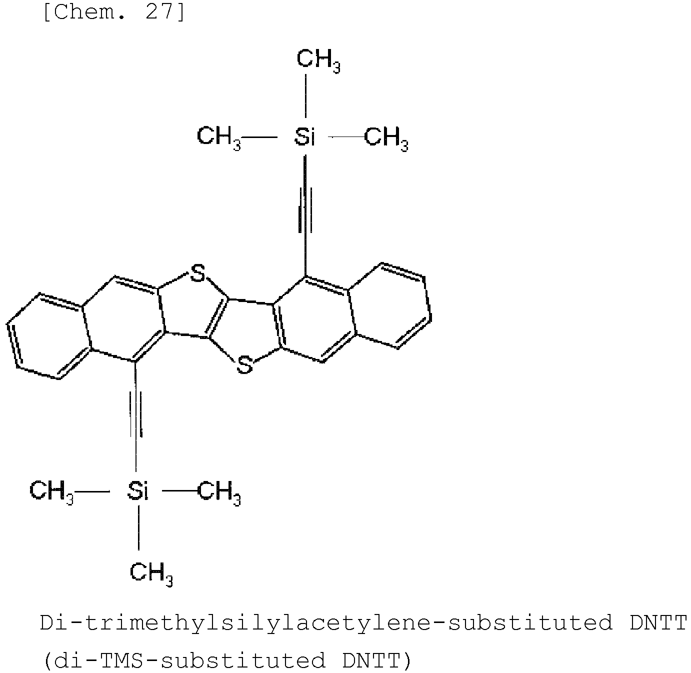

The ¹H-NMR and MS results of the obtained di-TMS-substituted DNTT are shown below.

¹H-NMR (500 MHz, CDCl3, 50°C): δ8.53 (d, J=8.3 Hz, 2H), 8.43 (s, 2H), 7.96 (d, J=8.3 Hz, 2H), 7.63 (dd, J=8.3 Hz, 8.3 Hz, 2H), 7.57 (dd, J=8.3 Hz, 8.3 Hz, 2H), 0.52 (s, 18H).

MS (m/z): 532.008 (positive ion observation) (Exact Mass: 532.111).

### <<Reference Example 10>>

The di-Br-substituted DNTT synthesized in Reference Example 1 was reacted with triethylsilylacetylene (TES) by the Negishi coupling method to synthesize di-TES-substituted DNTT.

To a 200-ml flask, 2.25 g (16.03 mmol) of triethylsilylacetylene (Mw=140.30, d=0.78 g/cm³) and 70 ml of toluene were added, and deaeration under reduced pressure and nitrogen purging were performed three times. After cooling to 0°C, 10.3 ml (16.68 mmol) of a hexane solution of n-BuLi was added and then, the temperature was raised to room temperature.

Thereafter, 4.31 g (17.06 mmol) of ZnCl₂ (TMEDA) (Mw=252.50), 1.0 g (2.00 mmol) of di-Br-substituted DNTT (Mw=498.25) and 704.8 mg (1.00 mmol) of Pd(PPh₃)₂Cl₂ (Mw=701.90) were added, and deaeration under reduced pressure and nitrogen purging were performed three times. The system was stirred at 100°C over 12 hours, thereby allowing the reaction to proceed.

By this reaction, di-triethylsilylacetylene-substituted DNTT (di-TES-substituted DNTT) (structural formula shown above, Mw=617.03, 321.40 mg, 0.52 mmol, yield: 26.0%) was obtained. The solubility of the obtained di-triethylsilylacetylene-substituted DNTT in chloroform was 0.10 wt%. Incidentally, DNTT used as a raw material was substantially not dissolved in chloroform.

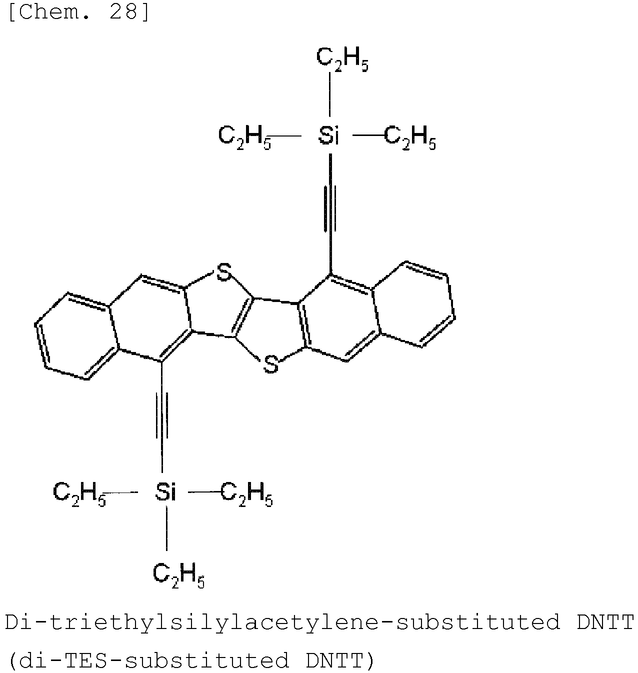

The ¹H-NMR and MS results of the obtained di-TES-substituted DNTT are shown below.

¹H-NMR (500 MHz, CDCl3, 50°C): δ8.56 (d, J=8.3 Hz, 2H), 8.42 (s, 2H), 7.96 (d, J=8.3 Hz, 2H), 7.63 (dd, J=8.3 Hz, 8.3 Hz, 2H), 7.57 (dd, J=8.3 Hz, 8.3 Hz, 2H), 1.25 (t, J=8.0 Hz, 18H), 0.94-0.98 (m, 12H).

MS (m/z): 616.057 (positive ion observation) (Exact Mass: 616.211).

### <<Reference Example 11>>

The di-Br-substituted DNTT synthesized in Reference Example 1 was reacted with thiophene by the Stille coupling method to synthesize di-thiophene-substituted DNTT.

To a 100-ml flask, 500 mg (1.00 mmol) of di-Br-substituted DNTT (Mw=498.25), 351.6 mg (0.501 mmol) of PdCl₂(PPh₃)₂ (Mw=701.90), 3.23g (8.64 mmol) of tributyl(2-thienyl)tin (Mw=373.18) and 35 ml of dry toluene were added, and nitrogen purging was performed three times. Thereafter, the system was stirred at 100°C all night.

After confirming that the peak of di-Br-substituted DNTT disappeared from MALDI, the system was cooled to room temperature. Chloroform and water were added, and the precipitate was filtered. The solvent was distilled of from the filtrate, and the peak of the target compound was confirmed by MS only from the filtrate. The black oily substance was washed twice by adding 10 ml of ether thereto, and the solid was filtered to obtain a dark green solid. Thereafter, the dark green solid was purified on a column, and the product was obtained as a yellow solid.

Di-thiophene-substituted DNTT (structural formula shown below, Mw=504.71, 158.80 mg, 0.31 mmol, yield: 31.4%) was obtained. The solubility of the obtained di-triethylsilylacetylene-substituted DNTT in chloroform was 0.13 wt%. Incidentally, DNTT used as a raw material was substantially not dissolved in chloroform.

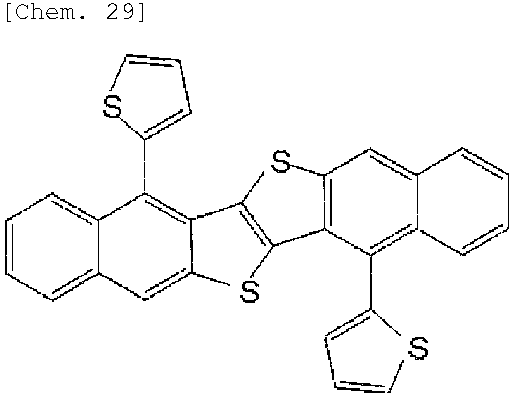

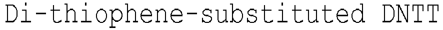

The ¹H-NMR and MS results of the obtained di-thiophene-substituted DNTT are shown below.

1H-NMR (500 MHz, CDCl3, 50°C): δ8.31 (s, 2H), 7.92 (d, J=6.5, 2H), 7.87 (d, J=7.0 Hz, 2H), 7.76 (dd, J=1.0 Hz, 1.0 Hz, 2H), 7.52-7.49 (m, 2H), 7.47-7.46 (m, 2H), 7.43-7.41 (m, 2H), 7.28-7.26 (m, 2H).

MS (m/z): 503.838 (positive ion observation) (Exact Mass: 504.013).

### <<Reference Example 12>>

The di-Br-substituted DNTT synthesized in Reference Example 1 was reacted with 1-bromodecane by the Negishi coupling method to synthesize di-decane-substituted DNTT.

Specifically, to a 10-ml flask, 548.2 mg (22.5 mmol) of magnesium was added, and deaeration under reduced pressure and nitrogen purging were performed three times. Thereafter, 2.5 ml of THF and 4.39 ml (21.3 mmol) of 1-bromodecane (Mw=221.18, d=1.07 g/cm³) were added, and refluxing was performed with stirring at 60°C for 1 hour.

After cooling to room temperature, 100 ml of toluene and 5.32 g (21.31 mmol) of ZnCl₂ (TMEDA) (Mw=252.50) were added, followed by stirring for 10 minutes. Thereafter, 1.25 g (2.50 mmol) of di-Br-substituted DNTT (Mw=498.25), 881.5 mg (1.25 mmol) of Pd(PPh₃)₂Cl₂ (Mw=701.90) and 77.5 ml of toluene were added, and deaeration under reduced pressure and nitrogen purging were performed three times. The system was stirred at 100°C over 12 hours, thereby allowing the reaction to proceed.

By this reaction, di-decane-substituted DNTT (structural formula shown below, Mw=620.99, 44.3 mg, 0.089 mmol, yield: 2.8%) was obtained. The solubility of the obtained di-decane-substituted DNTT in chloroform was 0.05 wt%. Incidentally, DNTT used as a raw material was substantially not dissolved in chloroform.

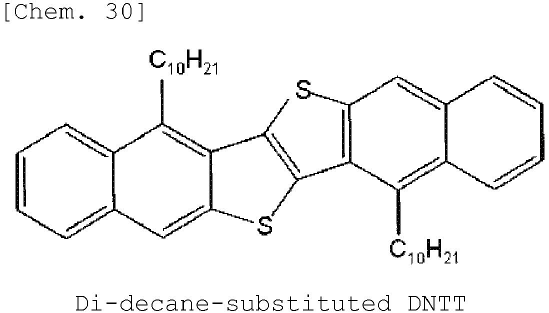

The ¹H-NMR and MS results of the obtained di-decane-substituted DNTT are shown below.

¹H-NMR (500 MHz, CDCl3, 50°C) : δ8.33 (s, 2H), 8.26 (d, J=8.30 Hz, 2H), 7.95 (d, J=8.3 Hz, 2H), 7.56 (dd, J=8.3 Hz, 8.3 Hz, 2H), 7.53 (dd, J=8.3 Hz, 8.3 Hz, 2H), 3.67-3.64 (m, 4H), 1.93-1.86 (m, 4H), 1.76-1.71 (m, 4H), 1.49-1.45 (m, 4H), 1.41-1.29 (m, 20H), 0.88 (t, J=6.8 Hz, 6H).

MS (m/z): 620.083 (positive ion observation) (Exact Mass: 620.288).

### DESCRIPTION OF REFERENCE NUMERALS

51, 81: Substrate
52, 82: Lower layer
53, 83: Upper layer

## Claims

1. An organic semiconductor solution containing an organic solvent, and a polymer and an organic semiconductor precursor which are dissolved in said organic solvent,
wherein the ratio of said polymer to the total of said polymer and said organic semiconductor precursor is 3 mass% or more, and
wherein said organic semiconductor precursor has a structure in which a dienophilic alkene is added in an eliminatable state through a double bond thereof to a benzene ring of an organic semiconductor compound of the following formula (I): 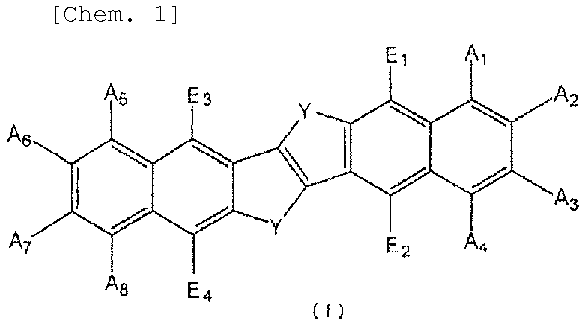 (wherein each of A₁ to A₈ and E₁ to E₄ is independently selected from the group consisting of hydrogen atom, halogen atoms, alkyl groups having from 1 to 20 carbon atoms, alkenyl groups having from 2 to 20 carbon atoms, alkynyl groups having from 2 to 20 carbon atoms, substituted or unsubstituted aromatic groups having from 4 to 20 carbon atoms, ketone groups having from 2 to 10 carbon atoms, amino groups having from 1 to 20 carbon atoms, amide groups having from 1 to 20 carbon atoms, imide groups having from 1 to 20 carbon atoms, sulfide groups having from 1 to 20 carbon atoms, and alkylsilylalkynyl groups having from 1 to 40 carbon atoms, two adjacent members of A₁ to A₈ may combine with each other to form a substituted or unsubstituted aromatic group having from 4 to 20 carbon atoms, and
Y is sulfur or selenium).

2. The organic semiconductor solution according to claim 1, wherein said dienophilic alkene is either one compound of the following formulae (II-1a) and (II-1b): 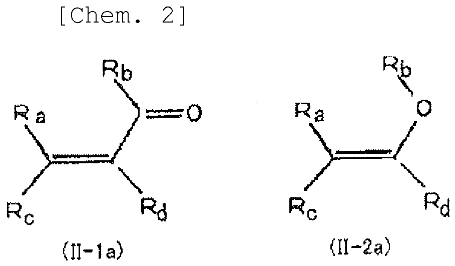 (wherein each of Rₐ, R_{b}, R_{c} and R_{d} is independently selected from the group consisting of a bond, hydrogen, halogens, hydroxyl group, amide groups, mercapto group, cyano group, alkyl groups having from 1 to 10 carbon atoms, alkenyl groups having from 2 to 10 carbon atoms, alkynyl groups having from 2 to 10 carbon atoms, alkoxy groups having from 1 to 10 carbon atoms, substituted or unsubstituted aromatic groups having from 4 to 10 carbon atoms, ester groups having from 1 to 10 carbon atoms, ether groups having from 1 to 10 carbon atoms, ketone groups having from 1 to 10 carbon atoms, amino groups having from 1 to 10 carbon atoms, amide groups having from 1 to 10 carbon atoms, imide groups having from 1 to 10 carbon atoms, and sulfide groups having from 1 to 10 carbon atoms,
Rₐ and R_{b} may combine with each other to form a ring, and
R_{c} and R_{d} may combine with each other to form a ring).

3. The organic semiconductor solution according to claim 1 to 2, wherein said dienophilic alkene has any one of the following formulae (II-1-1) to (II-2-3) : 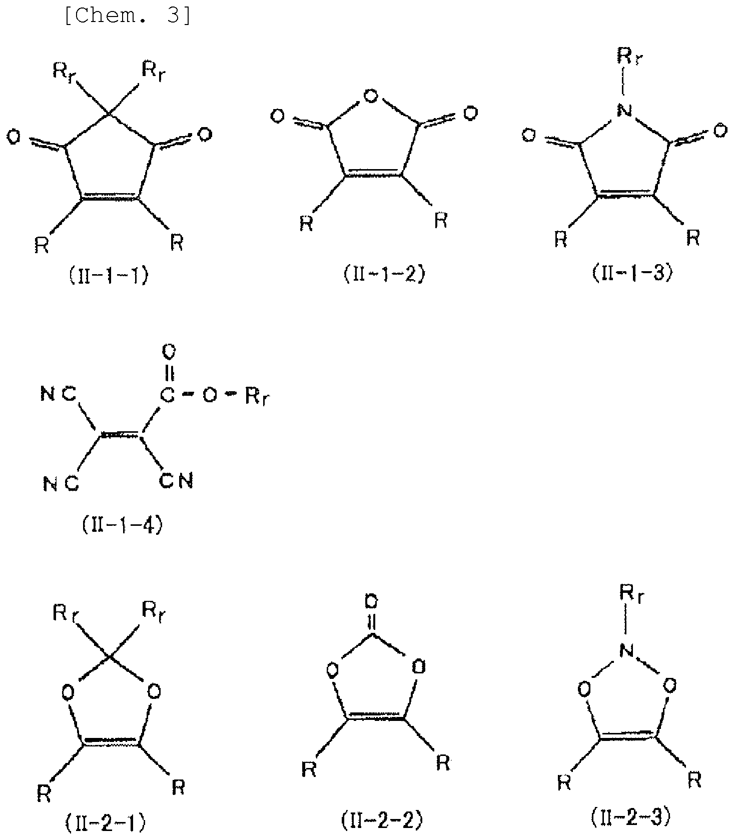 (wherein each of R and Rᵣ is independently selected from the group consisting of hydrogen, halogens, hydroxyl group, amide groups, mercapto group, cyano group, alkyl groups having from 1 to 10 carbon atoms, alkenyl groups having from 2 to 10 carbon atoms, alkynyl groups having from 2 to 10 carbon atoms, alkoxy groups having from 1 to 10 carbon atoms, substituted or unsubstituted aromatic groups having from 4 to 10 carbon atoms, ester groups having from 1 to 10 carbon atoms, ether groups having from 1 to 10 carbon atoms, ketone groups having from 1 to 10 carbon atoms, amino groups having from 1 to 10 carbon atoms, amide groups having from 1 to 10 carbon atoms, imide groups having from 1 to 10 carbon atoms, and sulfide groups having from 1 to 10 carbon atoms).

4. The organic semiconductor solution according to claim 3, wherein said dienophilic alkene has the following formula (II-1-3):

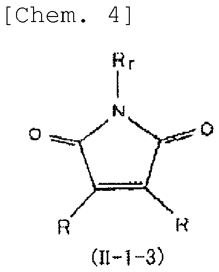

5. The organic semiconductor solution according to claim 4, wherein said organic semiconductor precursor has the following formula (1-1-1): 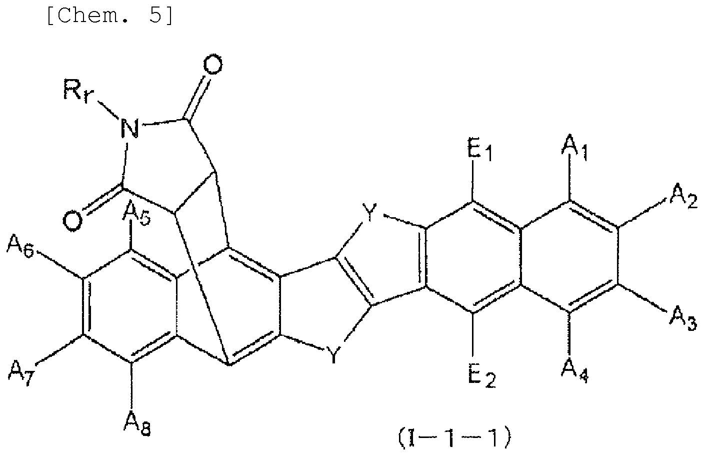 (wherein each of A₁ to A₈, E₁ and E₂ is independently selected from the group consisting of hydrogen atom, halogen atoms, alkyl groups having from 1 to 20 carbon atoms, alkenyl groups having from 2 to 20 carbon atoms, alkynyl groups having from 2 to 20 carbon atoms, substituted or unsubstituted aromatic groups having from 4 to 20 carbon atoms, ketone groups having from 2 to 10 carbon atoms, amino groups having from 1 to 20 carbon atoms, amide groups having from 1 to 20 carbon atoms, imide groups having from 1 to 20 carbon atoms, sulfide groups having from 1 to 20 carbon atoms, and alkylsilylalkynyl groups having from 1 to 40 carbon atoms, two adjacent members of A₁ to A₈ may combine with each other to form a substituted or unsubstituted aromatic group having from 4 to 20 carbon atoms,
each Rᵣ is independently selected from the group consisting of hydrogen, halogens, hydroxyl group, amide groups, mercapto group, cyano group, alkyl groups having from 1 to 10 carbon atoms, alkenyl groups having from 2 to 10 carbon atoms, alkynyl groups having from 2 to 10 carbon atoms, alkoxy groups having from 1 to 10 carbon atoms, substituted or unsubstituted aromatic groups having from 4 to 10 carbon atoms, ester groups having from 1 to 10 carbon atoms, ether groups having from 1 to 10 carbon atoms, ketone groups having from 1 to 10 carbon atoms, amino groups having from 1 to 10 carbon atoms, amide groups having from 1 to 10 carbon atoms, imide groups having from 1 to 10 carbon atoms, and sulfide groups having from 1 to 10 carbon atoms, and
Y is sulfur or selenium).

6. The organic semiconductor solution according to any one of claims 1 to 5, wherein the ratio of said polymer to the total of said polymer and said organic semiconductor compound is 90 mass% or less.

7. The organic semiconductor solution according to any one of claims 1 to 6, wherein the repeating unit of said polymer has a conjugated double bond and/or an aromatic ring.

8. The organic semiconductor solution according to any one of claims 1 to 7, wherein the repeating unit of said polymer does not contain an element other than carbon, hydrogen and a halogen.

9. The organic semiconductor solution according to any one of claims 1 to 8, wherein said polymer is an amorphous polymer selected from the group consisting of polycarbonate, polystyrene, acrylic resin, methacrylic resin, polyvinyl chloride, polyphenylene ether and polysulfone.

10. The organic semiconductor solution according to claim 9, wherein said amorphous polymer is a styrene-based polymer having a benzene ring moiety in the repeating unit.

11. The organic semiconductor solution according to claim 9, wherein said amorphous polymer is a polycarbonate-based polymer having a benzene ring moiety and a carbonate group in the repeating unit.

12. The organic semiconductor solution according to claim 9, wherein said amorphous polymer is an acrylic polymer composed of a polymer of an acrylic acid ester or a methacrylic acid ester.

13. A method for producing an organic semiconductor film, comprising:
coating the organic semiconductor solution according to any one of claims 1 to 9 on a substrate to produce a green film, and
applying light irradiation and/or heating to said green film to eliminate and remove said dienophilic alkene from said precursor and obtain a semiconductor film formed of said polymer and said organic semiconductor compound of formula (I).

14. An organic semiconductor film formed of a polymer and an organic semiconductor compound,
wherein the ratio of said polymer to the total of said polymer and said organic semiconductor compound is 3 mass% or more,
wherein said organic semiconductor compound has the following formula (I), and
wherein said organic semiconductor film satisfies at least one of the following conditions (i) to (iii) : 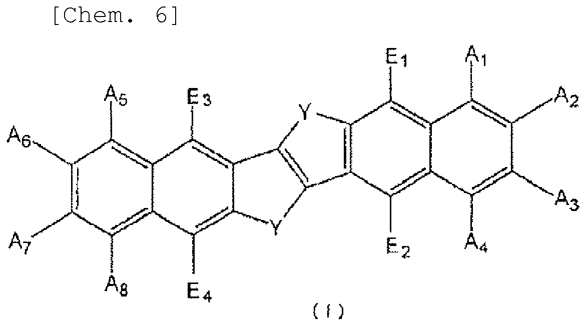 (wherein each of A₁ to A₈ and E₁ to E₄ is independently selected from the group consisting of hydrogen atom, halogen atoms, alkyl groups having from 1 to 20 carbon atoms, alkenyl groups having from 2 to 20 carbon atoms, alkynyl groups having from 2 to 20 carbon atoms, substituted or unsubstituted aromatic groups having from 4 to 20 carbon atoms, ketone groups having from 2 to 10 carbon atoms, amino groups having from 1 to 20 carbon atoms, amide groups having from 1 to 20 carbon atoms, imide groups having from 1 to 20 carbon atoms, sulfide groups having from 1 to 20 carbon atoms, and alkylsilylalkynyl groups having from 1 to 40 carbon atoms, two adjacent members of A₁ to A₈ may combine with each other to form a substituted or unsubstituted aromatic group having from 4 to 20 carbon atoms, and
Y is sulfur or selenium);
(i) the film has first and second layers stacked one on another, both of said first and second layers have said organic semiconductor compound, and the mass fraction of said organic semiconductor compound in said first layer is higher than the mass fraction of said organic semiconductor compound in said second layer,
(ii) the film further contains an organic semiconductor precursor, and said organic semiconductor precursor has a structure in which a dienophilic alkene is added in an eliminatable state through a double bond thereof to a benzene ring of the organic semiconductor compound of formula (I), and
(iii) the film contains a crystal of said organic semiconductor compound having a long axis diameter of more than 20 µm.

15. The organic semiconductor film according to claim 14, satisfying at least said (i).

16. The organic semiconductor film according to claim 14, satisfying at least said (ii).

17. The organic semiconductor film according to claim 14, satisfying at least said (iii).

18. The organic semiconductor film according to any one of claims 14 to 17, wherein the ratio of the polymer to the total of the polymer, the organic semiconductor and the optional organic semiconductor precursor is 50 mass% or less.

19. The organic semiconductor film according to any one of claims 14 to 18,
having a diffraction peak in the in-plane XRD observation, and
having a diffraction peak at 2θ of around 5.5° in the out-of-plane XRD observation.

20. The organic semiconductor film according to claim 19, having a diffraction peak at 2θ of around 18°, around 23° and around 27° in the in-plane XRD observation.

21. An organic semiconductor device, having the organic semiconductor film according to any one of claims 14 to 20.

22. The organic semiconductor device according to claim 21, which is a thin-film transistor having a source electrode, a drain electrode, a gate electrode, a gate insulating film and said organic semiconductor film, wherein said source electrode and said drain electrode are insulated from said gate electrode by said gate insulating film and the current flowing through said organic semiconductor from said source electrode to said drain electrode is controlled by the voltage applied to said gate electrode.
